# EUROPEAN PATENT SPECIFICATION

(11) **EP 4 420 661 B1**
(45) Date of publication and mention of the grant of the patent: **26.03.2025**
(21) Application number: 23382170.1
(22) Date of filing: 24.02.2023
(51) Int. Cl.: A61K 31/427, A61P 7/02, A61P 9/10, A61K 45/06

(54) **SMALL MOLECULES TARGETING GLYCOPROTEIN VI AS ANTIPLATELET AGENTS FOR USE IN THE PREVENTION AND/OR TREATMENT OF THROMBOSIS AND ASSOCIATED PATHOLOGIES**
GEGEN GLYCOPROTEIN VI GERICHTETE KLEINE MOLEKÜLE ALS THROMBOZYTENAGGREGATIONSHEMMER ZUR VERWENDUNG BEI DER VORBEUGUNG UND/ODER BEHANDLUNG VON THROMBOSE UND ZUGEHÖRIGEN ERKRANKUNGEN
PETITES MOLÉCULES CIBLANT LA GLYCOPROTÉINE VI EN TANT QU'AGENTS ANTIPLAQUETTAIRES POUR UNE UTILISATION DANS LA PRÉVENTION ET/OU LE TRAITEMENT DE LA THROMBOSE ET DE PATHOLOGIES ASSOCIÉES

(43) Date of publication of application: 28.08.2024
(73) Proprietor: Universidade de Santiago de Compostela, 15782 Santiago de Compostela (ES)
(72) Inventor: GARCÍA ALONSO, Ángel, 15782 Santiago de Compostela (ES); DOMÍNGUEZ MEDINA, Eduardo, 15782 Santiago de Compostela (ES); LOZA GARCÍA, María Isabel, 15782 Santiago de Compostela (ES); CARRACEDO ÁLVAREZ, Angel María, 15782 Santiago de Compostela (ES); CORREIA PINTO CARVALHO DE MATOS, María Joâo, 15782 Santiago de Compostela (ES); IZQUIERDO BERJANO, Irene, 15782 Santiago de Compostela (ES)
(74) Representative: Manuel Illescas y Asociados, S.L.

(56) References cited:
- WO-A1-2018/237084
- JP-A- 2019 064 976
- KR-A- 20190 026 154
- US-A1- 2017 174 699
- COREY VICTORIA C. ET AL: "A broad analysis of resistance development in the malaria parasite", NATURE COMMUNICATIONS, vol. 7, no. 1, 15 June 2016 (2016-06-15), pages 1 - 9, XP093054573, Retrieved from the Internet <URL:https://www.nature.com/articles/ncomms11901.pdf> DOI: 10.1038/ncomms11901
- LOCKYER ET AL: "GPVI-deficient mice lack collagen responses and are protected against experimentally induced pulmonary thromboembolism", THROMBOSIS RESEARCH, ELSEVIER, AMSTERDAM, NL, vol. 118, no. 3, 1 January 2006 (2006-01-01), pages 371 - 380, XP005566491, ISSN: 0049-3848

## Description

### FIELD OF THE INVENTION

The present invention belongs to the pharmaceutical sector and, more in particularly, refers to a family of molecules which act as ligands of glycoprotein VI (GPVI). Since the molecules described in present invention block GPVI-mediated platelet activation they are, therefore, useful as platelet aggregation inhibitors. Accordingly, present invention refers to a family of ligands of GPVI, as well as compositions comprising the same, for use in the prevention and/or treatment of thrombosis, as well as, pathologies associated to thrombus formation, such as ischemic stroke, atherosclerosis, coronary artery disease or myocardial infarction.

### BACKGROUND OF THE INVENTION

Platelets or thrombocytes are essential for hemostasis or bleeding prevention and also play other roles in innate immunity and in participating in many inflammatory processes. Hemostasis is the process by which platelets are activated to form aggregates or clumps with red blood cells and fibrin protein, resulting in thrombus formation. A thrombus is, in principle, the appropriate response of the organism to a bleeding injury but it may be harmful when is formed in healthy blood vessels, obstructing the circulatory system,

In fact, thrombus formation on an intact endothelium is prevented by nitric oxide, prostacyclin and CD39. However, platelet malfunctioning or hyperreactivity represents an important risk to develop thrombosis and ischemic events.

Antiplatelet drugs may inhibit the process involved in platelet activation, reversibly or irreversibly. Over the last decades, antiplatelet drugs, have significantly reduced morbidity and mortality associated with arterial thrombosis. Their pharmacological characteristics, including pharmacokinetic/pharmacodynamics profiles, have been extensively studied, and a significant number of clinical trials assessing their efficacy and safety in various clinical settings have established antithrombotic efficacy.

The currently available antiplatelet drugs act by preventing the formation of secondary messengers (COX-1 inhibitor), by interacting with intracellular signaling pathways (PDE inhibitors and the PGI2 analogue), or by blocking membrane receptors (P2Y12 receptor antagonists and the PAR1 antagonist), or by inhibiting platelet aggregation (GPllbllla inhibitors) per se.

However, the administration, often chronic, of antiplatelet therapies is associated with an inherent risk of bleeding. Given that bleeding is associated with adverse cardiovascular outcomes and mortality, there is an unmet clinical need to develop novel antiplatelet therapies that inhibit thrombosis while maintaining hemostasis and reduce adverse bleeding events.

Many of platelet's roles are regulated by glycoprotein VI (GPVI) and C-type lectin receptor 2 (CLEC-2), which signal through an immunoreceptor tyrosine-based activation motif (ITAM). Receptors with an immunoreceptor tyrosine-based activation motif (ITAM) are important in supporting the signaling and activation of the integrins that are essential to platelet function. In fact, GPVI represents an attractive target to manage platelet function since it is only expressed on platelets and platelet precursor cells in bone marrow (megakaryocytes). Binding of GPVI to collagen, one of the most important thrombogenic components, induces receptor clustering and subsequent platelet activation [Lockyer S. et al (2006)].

The patent (KR 20190026154A) describes compounds to be used as inhibitors of binding between the TRAF4 protein and one or more of GPlb and GPVI for treating thrombosis.

Since GPVI plays a critical role in platelet activation and thrombus initiation, as well as in prevention of inflammation-induced hemorrhage, it represents therefore an attractive target over current antiplatelet therapies in which bleeding may be a problem.

### DESCRIPTION OF THE INVENTION

The present invention refers to a family of compounds of formula (I), herein described, which act as GPVI ligands and are useful as drug-like inhibitors of Ca+2 mobilization and platelet aggregation, following GPVI activation. In this connection, said family of compounds of formula (I) described herein are useful, accordingly, as antiplatelet drugs preventing platelet aggregation and thrombus formation, for the prevention and/or treatment of thrombosis and, pathologies associated to thrombus formation, such as ischemic stroke, atherosclerosis, coronary artery disease or myocardial infarction, while preventing adverse bleeding events, due to its administration. The family of compounds of formula (I), herein described is also useful as pharmacological tool, such as tools for *in-vitro* inhibition studies interfering with platelet activation mediated by GPVI. In particular, present invention refers to a compound of formula (I), or a pharmaceutically acceptable salt or solvate thereof: 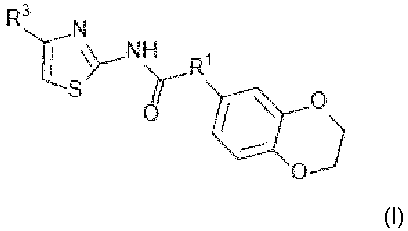 wherein R¹ is selected from a group consisting of -CH₂- or a group R²: 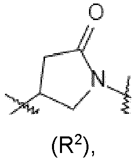 wherein R² is attached to the 2,3-dihydrobenzo[b][1,4]dioxine by the nitrogen atom of the pyrrolidin-2-one ring; and wherein R³ is selected from the group consisting of a phenyl substituted with one or more groups R⁴, a naphthyl group substituted with one or more groups R⁴, a 1,2,3,4-tetrahydronapht-6-yl group substituted with one or more groups R⁴ and a 2-oxo-1,2,3,4-tetrahydroquinolin-6-yl group; and wherein R⁴ is H or a halogen; for use as an antiplatelet medicament; or for use as a platelet aggregation inhibiting medicament; and therefore, for use as a GPVI inhibitor in the prevention and/or treatment of thrombosis, as well as, pathologies associated to thrombus formation, such as ischemic stroke, atherosclerosis, coronary artery disease or myocardial infarction, while preventing adverse bleeding events.

Accordingly, present invention refers to a compound of formula (I), as described herein, for use in the prevention and/or treatment of diseases mediated by GPVI ligands, as well as diseases that respond to inhibitors of Ca+2 mobilization and platelet aggregation following GPVI activation. In this connection, present invention also refers to a compound of formula (I), as described herein, for use as an antiplatelet drug, preventing platelet aggregation and thrombus formation, and therefore, for use in the prevention and/or treatment of diseases that respond to inhibitors of GPVI, in particular, thrombosis, as well as, pathologies associated to thrombus formation, such as ischemic stroke, atherosclerosis, coronary artery disease or myocardial infarction, while preventing adverse bleeding events. Additionally or alternatively, the present invention concerns an *in-vitro* use of a compound of formula (I) as a pharmacologic tool.

For example, the compound of formula (I) as described herein may be used in *in-vitro* inhibition studies interfering platelet activation mediated by GPVI.

The term "pharmaceutically acceptable salt" refers to any pharmaceutically acceptable salt, which upon administration to the patient is capable of providing (directly or indirectly) a compound as described herein. Such salts preferably are acid addition salts with physiologically acceptable organic or inorganic acids, or alkali addition salts with acceptable organic or inorganic bases. Examples of the acid addition salts include mineral acid addition salts such as, for example, hydrochloride, hydrobromide, hydroiodide, sulphate, nitrate, phosphate, and organic acid addition salts such as, for example, lactate, acetate, trifluoroacetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium and ammonium salts, and organic alkali salts such as, for example, ethylenediamine, ethanolamine, N,N-dialkylenethanolamine, triethanolamine and basic aminoacids salts. However, it will be appreciated that non-pharmaceutically acceptable salts also fall within the scope of the invention since those may be useful in the preparation of pharmaceutically acceptable salts. Procedures for salt formation are conventional in the art.

The term "pharmaceutically acceptable solvate" in accordance with this invention should be understood as meaning any form of the active compound in accordance with the invention in which said compound is bonded by a non-covalent bond to another molecule (normally a polar solvent), including especially hydrates and alcoholates.

Another preferred aspect of present invention refers to a pharmaceutical composition comprising at least one compound of formula (I), or a pharmaceutically acceptable salt or solvate thereof as disclosed herein; and at least one pharmaceutically acceptable excipient, for use as an antiplatelet drug, preventing platelet aggregation and thrombus formation, and therefore, for use as a GPVI inhibitor in the prevention and/or treatment of thrombosis, as well as, pathologies associated to thrombus formation, such as ischemic stroke, atherosclerosis, coronary artery disease or myocardial infarction, while preventing adverse bleeding events.

The characteristics of the compounds of formula (I) as ligands of GPVI, makes them, accordingly, inhibitors of platelet aggregation and, therefore, useful as well as in the prevention and/or treatment of pathologies associated to thrombus formation, such as ischemic stroke, atherosclerosis, coronary artery disease or myocardial infarction.

The term "thrombosis" refers, for the purposes of the present invention, to the formation of a blood clot within blood vessels that limits the flow of blood therein and resulting in possible damage (vessel rupture, low oxygen supply to a tissue or organ, etc.). Thrombus formation may occur in veins (blood vessels carrying blood from different parts of the body to the heart) or arteries (blood vessels supplying blood, rich in oxygen, from the heart to different parts of the body). Therefore, the term thrombosis as used herein refers to venous thrombosis and arterial thrombosis, preferably arterial thrombosis. Thrombosis accounts for the most common causes of death in developed countries. This mortality depends on location and acuity of the thrombus formed, with myocardial infarction and ischemic stroke accounting for the highest proportion of thrombosis-associated deaths (Ashorobi et al., 2021).

For the purposes of the present invention the term "arterial thrombosis" is used in an equivalent manner to the term "atherothrombosis" and refers to the formation of a blood clot within arteries, limiting the flow of blood therein. Platelets play a significant role in the development of arterial thrombosis compared to venous thrombosis, and this explains the importance of antiplatelet agents in the prevention and treatment of arterial thrombosis. Arterial thrombosis, or atherothrombosis, may initiate by the accumulation of lipid plaques in the arterial wall, provoking chronic inflammatory cells and platelet activation, as can be seen with coronary artery disease. The initial lipid plaques evolve into fibrous plaques which may rupture and lead to the release of additional pro-coagulating factors. This process is then called "atherosclerosis" for the purposes of the present invention. Atherosclerosis allows the activation of platelets, causing adhesion and aggregation, which leads to the formation of a clot. The occlusion of vessels due to atherosclerosis and thrombin formation in the coronary arteries of the heart may lead to ischemic heart disease and myocardial infarction. Additionally, the term "ischemic stroke" refers, for the purposes of the present invention, to the onset of a stroke or cerebrovascular accident which is due a thrombotic event that causes a decrease in blood flow to the brain.

Finally, for the purposes of the present invention the term "coronary artery disease" refers to a pathology which involves atherosclerotic plaque formation in the vessel lumen. This leads to impairment in blood flow and thus oxygen delivery, in particular, to the myocardium, and may result in a myocardial infarction, which refers, accordingly, to a decrease or complete cessation of in blood flow to the myocardium.

Accordingly, present invention also refers to a compound of formula (I), or a pharmaceutically acceptable salt or solvate thereof, or a pharmaceutical composition comprising at least one compound of formula (I), or a pharmaceutically acceptable salt or solvate thereof, as disclosed herein; for use in the prevention and/or treatment of diseases that respond to inhibitors of GPVI, and accordingly, for use as a GPVI inhibitor in the prevention and/or treatment of arterial thrombosis and atherothrombosis, as well as, pathologies associated to thrombus formation, wherein said pathologies associated to thrombus formation are ischemic stroke, atherosclerosis, coronary artery disease or myocardial infarction.

Yet another aspect of present invention refers to the use of a compound of formula (I), or a pharmaceutically acceptable salt or solvate thereof, or the use of a pharmaceutical composition comprising at least one compound of formula (I), or a pharmaceutically acceptable salt or solvate thereof, as disclosed herein, for manufacturing a medicament for preventing platelet aggregation and thrombus formation, in particular by inhibiting GPVI, and therefore, a medicament for prevention and/or treatment of thrombosis, as well as, pathologies associated to thrombus formation, such as ischemic stroke, atherosclerosis, coronary artery disease or myocardial infarction, while preventing adverse bleeding events.

Moreover, present invention also refers to the use of a compound of formula (I), or a pharmaceutically acceptable salt or solvate thereof, or the use of a pharmaceutical composition comprising at least one compound of formula (I), or a pharmaceutically acceptable salt or solvate thereof, as disclosed herein, for manufacturing a medicament for preventing and/or treating ischemic stroke, atherosclerosis, coronary artery disease or myocardial infarction.

Yet another aspect of present invention refers to the *in-vitro* use of a compound of formula (I), or a pharmaceutically acceptable salt or solvate thereof, or the *in-vitro* use of a pharmaceutical composition comprising at least one compound of formula (I), or a pharmaceutically acceptable salt or solvate thereof, as disclosed herein, as a pharmacologic tool. The term "pharmacologic tool" refers to any suitable pharmacologic tool. Specifically, the compounds of formula (I) as herein described are GPVI inhibitors. By inhibiting GPVI, platelet activation is interfered with. Thus, these compounds can be used in *in-vitro* test systems for the detection of GPVI-mediated platelet activation. For example, in a test system in which platelet activation is observed in the absence of a compound of formula I as disclosed herein, if then a reduction of platelet activation is observed upon addition of a compound of formula I as disclosed herein, at least part of the observed platelet activation is due to GPVI. Thus, the tool may be a tool to study the *in-vitro* inhibition by interfering platelet activation mediated by GPVI.

For the purposes of present invention, the term "comprises" may be replaced by any of the terms "consists of", or "consists essentially of". Accordingly, "comprises" may refer to a group of features A, B and C, which additionally may include other features, such as E and D, with the condition that said features do not render the claim unworkable, but said term "comprises" also includes the situation in which the group of features "consists of" or "consists essentially" of A, B and C.

For the purposes of present invention, an effective amount of the compounds disclosed herein, when said effective amount is referred to a therapeutic use of said compounds, is that which provides a therapeutic effect in the subject or an objectively identifiable improvement in the health condition of said subject as noted by the clinician or other qualified observer.

In a preferred embodiment of the invention, R¹ is -CH₂- and R³ is selected from the group consisting of a phenyl substituted with one or more groups R⁴, a naphthyl group substituted with one or more groups R⁴, a 1,2,3,4-tetrahydronapht-6-yl group substituted with one or more groups R⁴ and a 2-oxo-1,2,3,4-tetrahydroquinolin-6-yl group; and wherein R⁴ is H or a halogen.

More preferably, R¹ is -CH₂- and R³ is a non-substituted phenyl group, a phenyl group substituted with one or more halogen groups, a naphthyl group, a 1,2,3,4-tetrahydronapht-6-yl group or a 2-oxo-1,2,3,4-tetrahydroquinolin-6-yl group. Even more preferably, R¹ is -CH₂- and R³ is a non-substituted phenyl group, a 4-chlorophenyl group, a 4-bromophenyl group, a naphthyl group, a 1,2,3,4-tetrahydronapht-6-yl group or a 2-oxo-1,2,3,4-tetrahydroquinolin-6-yl group.

In another preferred embodiment of the invention, R¹ is a group R²: 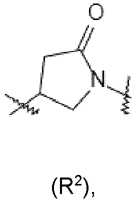 wherein R² is attached to the 2,3-dihydrobenzo[b][1,4]dioxine by the nitrogen atom of the pyrrolidin-2-one ring; and R³ is selected from the group consisting of a phenyl substituted with one or more groups R⁴, a naphthyl group substituted with one or more groups R⁴, a 1,2,3,4-tetrahydronapht-6-yl group substituted with one or more groups R⁴ and a 2-oxo-1,2,3,4-tetrahydroquinolin-6-yl group; and wherein R⁴ is H or a halogen.

More preferably, R¹ is a group R², as disclosed herein, and R³ is a non-substituted phenyl group, a phenyl group substituted with one or more halogen groups, a naphthyl group, a 1,2,3,4-tetrahydronapht-6-yl group or a 2-oxo-1,2,3,4-tetrahydroquinolin-6-yl group. Even more preferably, R¹ is a group R², as disclosed herein, and R³ is a 2-oxo-1,2,3,4-tetrahydroquinolin-6-yl group.

In a more preferred embodiment of the invention, the compound of formula (I) is selected from the group consisting of Iₐ, I_{b}, I_{c}, I_{d}, Iₑ, I_{f} and Iₕ, or a pharmaceutically acceptable salt or solvate thereof:

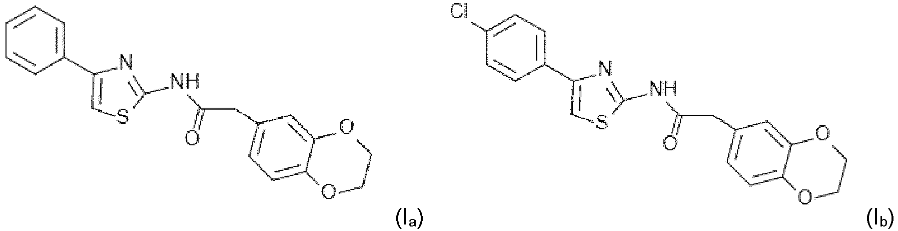

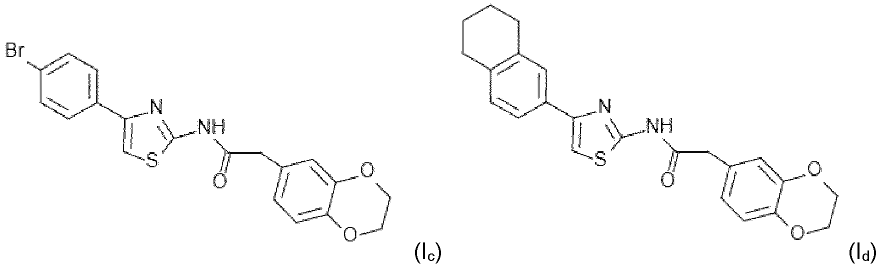

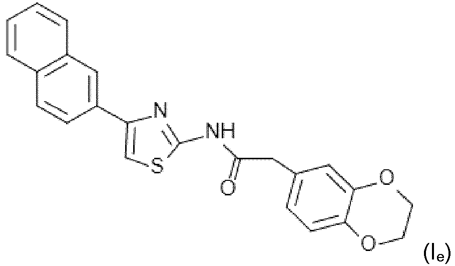

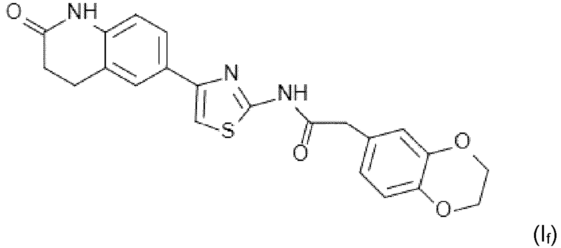

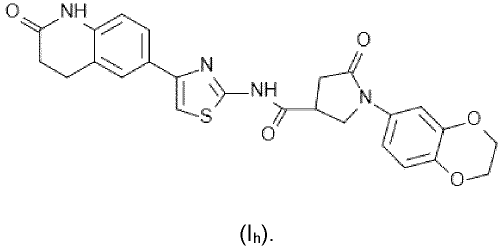

Advantageously, R¹ is -CH₂- and R³ is a non-substituted phenyl group and the compound of formula (I) is the compound (Iₐ), or R¹ is -CH₂- and R³ is a 4-chlorophenyl group and the compound of formula (I) is the compound (I_{b}), or R¹ is -CH₂- and R³ is a 4-bromophenyl group and the compound of formula (I) is the compound (I_{c}), or R¹ is -CH₂- and R³ is a naphthyl group and the compound of formula (I) is the compound (Iₑ), or R¹ is -CH₂- and R³ is a 1,2,3,4-tetrahydronapht-6-yl group and the compound of formula (I) is the compound (I_{d}), or R¹ is - CH₂- and R³ is a 2-oxo-1,2,3,4-tetrahydroquinolin-6-yl group and the compound of formula (I) is the compound (I_{f}), or R¹ is a group R², as disclosed herein, and R³ is a 2-oxo-1,2,3,4-tetrahydroquinolin-6-yl group and the compound of formula (I) is the compound (Iₕ).

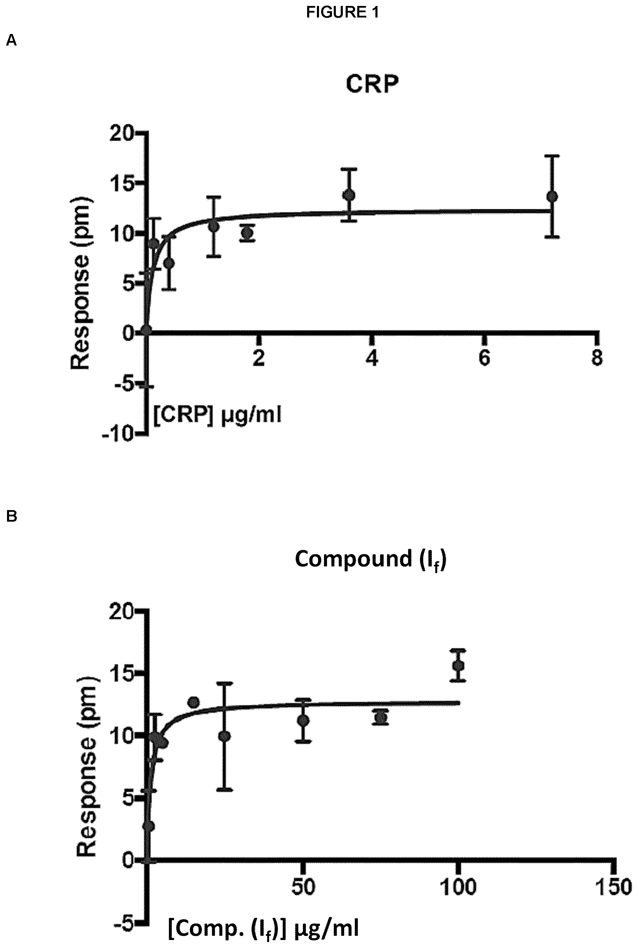

As seen in the examples that form part of present description, and in particular in Figure 1 and Table 1, the compounds of formula (I) disclosed herein act as ligands of GPVI. For instance, compound (If) shows a similar robust dose-dependent response upon interaction with GPVI, in a very similar manner as its natural ligand, the collagen related peptide (CRP).

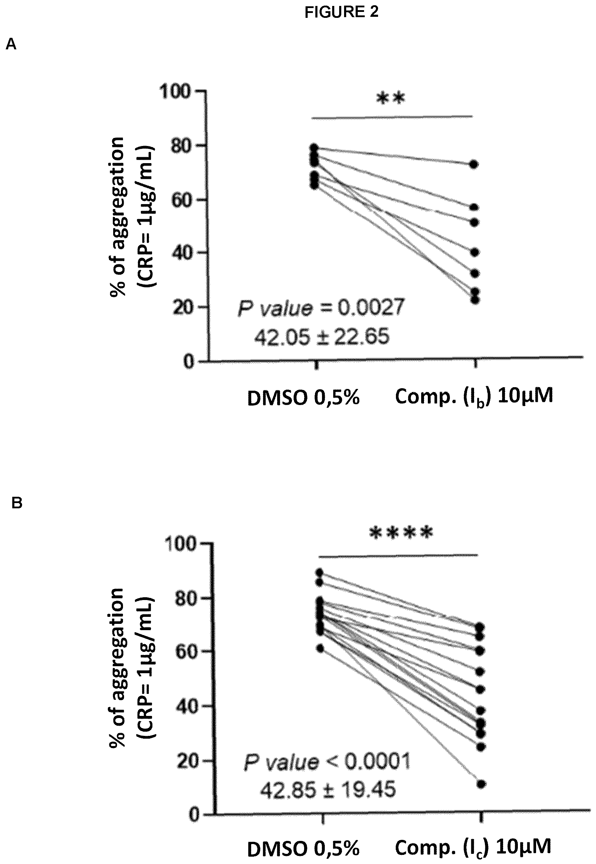

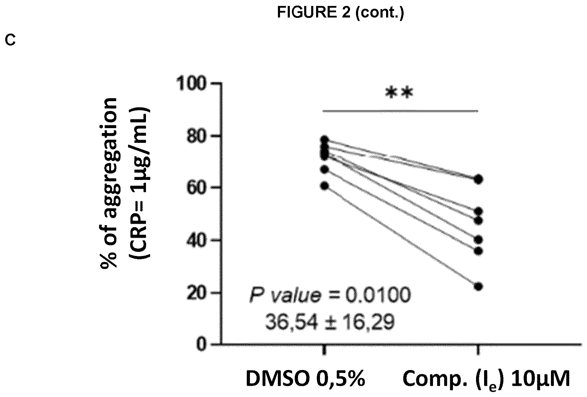

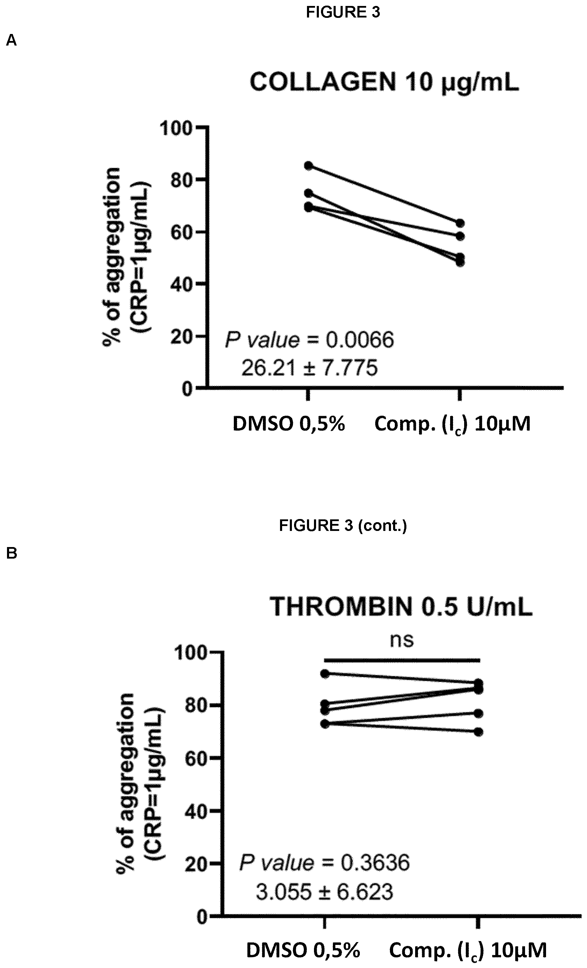

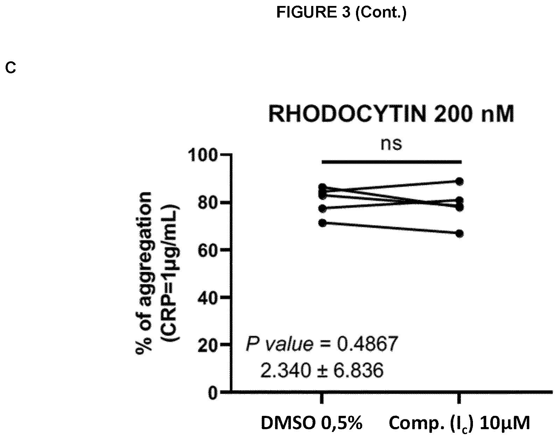

Moreover, as seen in the examples disclosed herein, the compounds of formula (I) for use according to present invention are capable of inhibiting platelet aggregation by inhibit calcium mobilization (as seen in Table 2 and Figure 2). However, the inhibition of platelet aggregation provided by the compounds of formula (I) disclosed herein is specifically induced by the GPVI natural ligand CRP, while aggregation was unchanged when other agonists such as rhodocytin or thrombin were used (see Figure 3).

This selectivity of the compounds of formula (I) as ligands of GPVI, makes them more specific inhibitors of platelet's aggregation in comparison with other compounds which may act as antithrombotic agents through other biological targets and, therefore, may cause a wider range of adverse effects, such as bleeding. The improved safety profile of the compounds of formula (I) in relation to adverse events, such as bleeding, potentially allows the administration of said compounds in a broader group of patients or in clinical situations where other antiplatelet agents may not be used due to their adverse effects (for instance, prior to surgery, or in patients where bleeding results in important health issues).

Preferably, the compound of formula (I) for use according to present invention, or the pharmaceutical compositions for use according to the present invention, as disclosed herein, may be administered prior to, in combination with, or subsequently after, administering an additional therapeutic agent for use in prevention and/or treatment of thrombosis , as well as, pathologies associated to thrombus formation, such as ischemic stroke, atherosclerosis, coronary artery disease or myocardial infarction, having additive or synergistic biological activities.

Also preferably, the pharmaceutical compositions disclosed herein may optionally comprise at least a second therapeutic agent for use in prevention and/or treatment of thrombosis such as arterial thrombosis, as well as, pathologies associated to thrombus formation, such as ischemic stroke, atherosclerosis, coronary artery disease or myocardial infarction, having additive or synergistic biological activities.

For the purposes of present invention, the term "therapeutic agent" should be taken as synonyms and mean a chemical entity which exerts therapeutic effects when administered to a patient.

More preferably, said additional therapeutic agent for use in prevention and/or treatment of thrombosis such as arterial thrombosis, as well as, pathologies associated to thrombus formation, such as ischemic stroke, atherosclerosis, coronary artery disease or myocardial infarction, is an antiplatelet drug. Even more preferably, said additional therapeutic agent for use in prevention and/or treatment of thrombosis such as arterial thrombosis, as well as, pathologies associated to thrombus formation, such as ischemic stroke, atherosclerosis, coronary artery disease or myocardial infarction, is selected from the group consisting of a cyclooxygenase inhibitor, an adenosine diphosphate receptor inhibitor, a phosphodiesterase inhibitor, protease-activated receptor-1 antagonist, a glycoprotein IIB/IIIA inhibitor, an adenosine reuptake inhibitor and a thromboxane inhibitor.

The compositions for use according to the invention may be formulated so as to provide quick, sustained, or delayed release of the compounds for use according to present invention after administration to the patient by employing procedures well known in the art.

Slow or extended-release delivery systems, including any of a number of biopolymers (biological-based systems), systems employing liposomes, colloids, resins, and other polymeric delivery systems or compartmentalized reservoirs, can be utilized with the compositions described herein to provide a continuous or long-term source of the therapeutic compounds disclosed herein.

As disclosed herein, the route of administration may be any route which effectively transports the compounds and compositions disclosed in present disclosure, to the appropriate or desired site of action, such as oral, nasal, topical, pulmonary, transdermal or parenteral, e. g., rectal, subcutaneous, intravenous, intraurethral, intramuscular, intranasal, ophthalmic solution or an ointment.

Preferably, the compound of formula (I) for use according to present invention, or the pharmaceutical compositions for use according to the present invention, as disclosed herein, may be administered orally, transdermally or parenterally.

The excipient included in the pharmaceutical compositions for use according to present invention, refers, for the purpose of present invention, to an inert ingredient such as, but not limited to, cosolvents, surfactants, oils, humectants or wetting agents, emulsifiers and suspending agents, sweetening or flavoring agents, preservatives, stabilizers and antioxidants.

Effective amounts of excipients are those amounts that are effective to obtain a pharmaceutically acceptable formulation in terms of solubility, biological activity, etc.

Such pharmaceutical compositions for use according to present invention may be included in a capsule, sachet, paper or other container. In making the compositions, conventional techniques for the preparation of pharmaceutical compositions may be used. For example, the compounds for use according to present invention may be mixed with an excipient, or diluted with an excipient, or enclosed within an excipient. When the excipient serves as a diluent, it may be solid, semi-solid, or liquid material that acts as a vehicle or medium for the active compound. The compounds for use according to present invention can be adsorbed on a granular solid container for example in a sachet. Said compositions may also include wetting agents, emulsifying and suspending agents, preserving agents, sweetening agents or flavoring agents, preservatives, stabilizers and antioxidants.

The pharmaceutical compositions can be sterilized and mixed, if desired, with emulsifiers, salts for influencing osmotic pressure, buffers and/or coloring substances, which do not deleteriously react with the compounds for use according to present invention. Any pharmacologically acceptable buffer may be used, such as, tris or phosphate buffers.

Some examples of suitable excipients are water, salt solutions, alcohols, polyethylene glycols, polyhydroxyethoxylated castor oil, peanut oil, olive oil, lactose, terra alba, sucrose, cyclodextrin, amylose, magnesium stearate, talc, gelatin, agar, pectin, acacia, stearic acid or lower alkyl ethers of cellulose, silicic acid, fatty acids, fatty acid amines, fatty acid monoglycerides and diglycerides, pentaerythritol fatty acid esters, polyoxyethylene, hydroxymethylcellulose, and polyvinylpyrrolidone. Similarly, the excipient or diluent may include any sustained release material known in the art, such as glyceryl monostearate or glyceryl distearate, alone or mixed with a wax.

**The** dosage of the compounds and compositions disclosed in accordance with the invention varies depending on the compound and the condition being treated for example the age, weight, and clinical condition of the recipient patient, as well as their use in combination with other therapeutic agents. Other factors include: the route of administration, the patient, the patient's medical history, the severity of the disease process, and the potency of the particular compound. **The** dose, or effective amount, should be sufficient to ameliorate symptoms or signs of the disease treated without producing unacceptable toxicity to the patient.

Accordingly, another preferred embodiment of the invention is the dosage scheme of the compounds and compositions for use according to present invention. **The** term "unit dosage form" refers to physically discrete units suitable as unitary dosages for subjects, each unit containing a predetermined quantity of active material calculated to produce the desired pharmaceutical effect in association with the required pharmaceutical excipient (diluent, carrier or vehicle). The specifications for the unit dosage forms of this invention are dictated by and dependent on (a) the unique characteristics of the compounds disclosed above herein and the particular effect to be achieved and (b) the limitations inherent in the art wherein said compounds are used in a subject in need thereof. Examples of unit dosage forms are tablets, capsules, pills, powder packets, wafers, suppositories, granules, cachets, teaspoonfuls, tablespoonfuls, dropperfuls, ampoules, vials, aerosols with metered discharges, segregated multiples of any of the foregoing, and other forms as herein described. The compounds compositions disclosed herein can be included in kits, which can contain one or more-unit dosage forms of the compositions, as well as other compositions comprising additional therapeutic agents, as disclosed herein, and instructions for use.

Accordingly, another aspect of present invention refers to a kit of parts comprising one or more-unit dosage forms of a pharmaceutical composition comprising at least one compound of formula (I), as disclosed herein, and at least one pharmaceutically acceptable excipient; and optionally, one or more unit dosage forms of another pharmaceutical composition comprising one or more additional therapeutic agent for use in prevention and/or treatment of thrombosis such as arterial thrombosis, as well as, pathologies associated to thrombus formation, such as ischemic stroke, atherosclerosis, coronary artery disease or myocardial infarction, as disclosed herein, and instructions for use.

The instructions refer to the mode of administration indicating that the unit dosage forms herein described, comprising the compounds and compositions disclosed in the present invention, may be administered one or more times daily, weekly, monthly, etc.

### BRIEF DESCRIPTION OF THE FIGURES

**FIG. 1****.** Interaction between GPVI and **(A)** collagen related peptide (CRP), the natural ligand for GPVI, or **(B)** compound (I_{f}), at different concentrations.
FIG. **2****.** Effect **of (A)** compound (I_{b}); **(B)** compound (I_{c}) and (C) compound (Iₑ) at a concentration 10 µM, in CRP-induced (1µg/mL) aggregation in human platelets. DMSO 0.5% was used as a control.
**FIG. 3****.** Inhibition of platelet aggregation by compound (I_{c}) in platelets exposed to (A) collagen, **(B)** thrombin and **(C)** rhodocytin. DMSO 0.5% was used as a control.

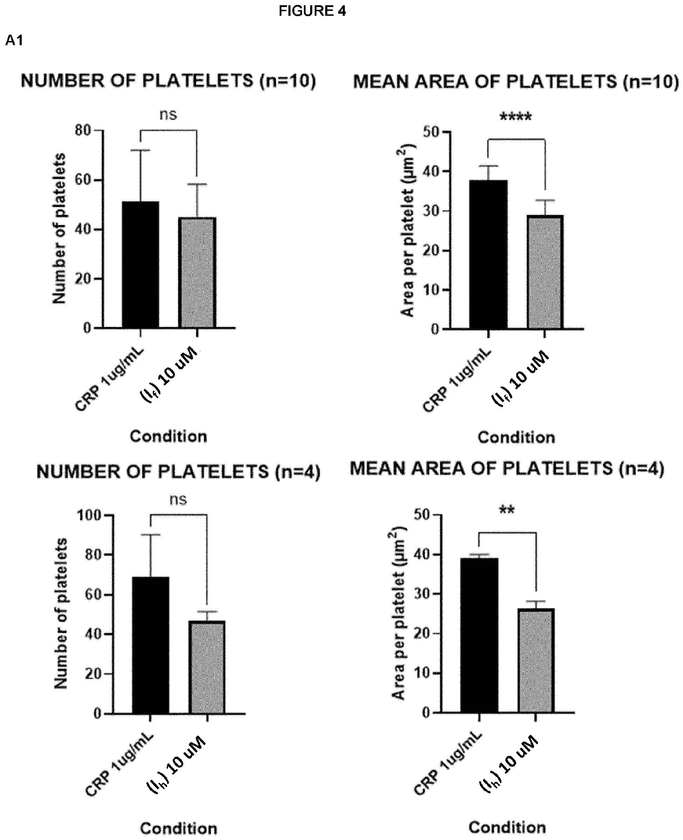

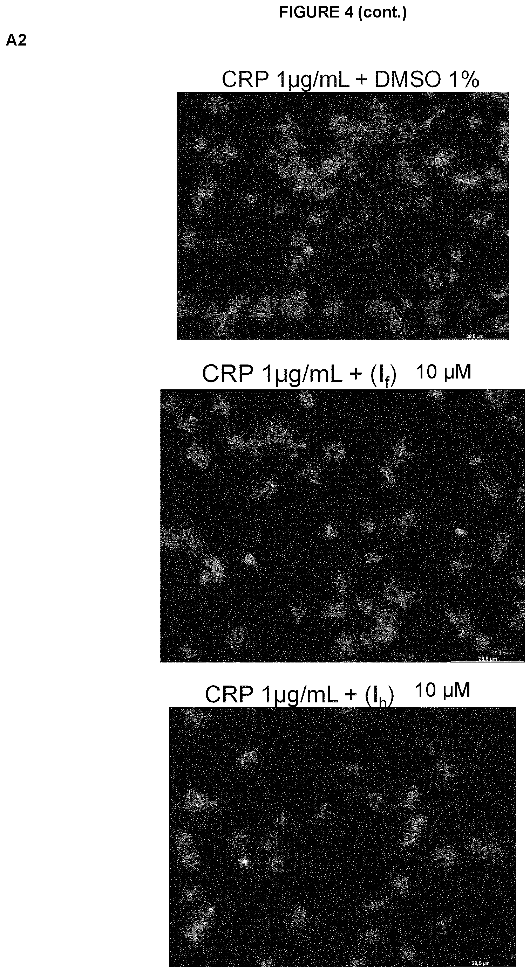

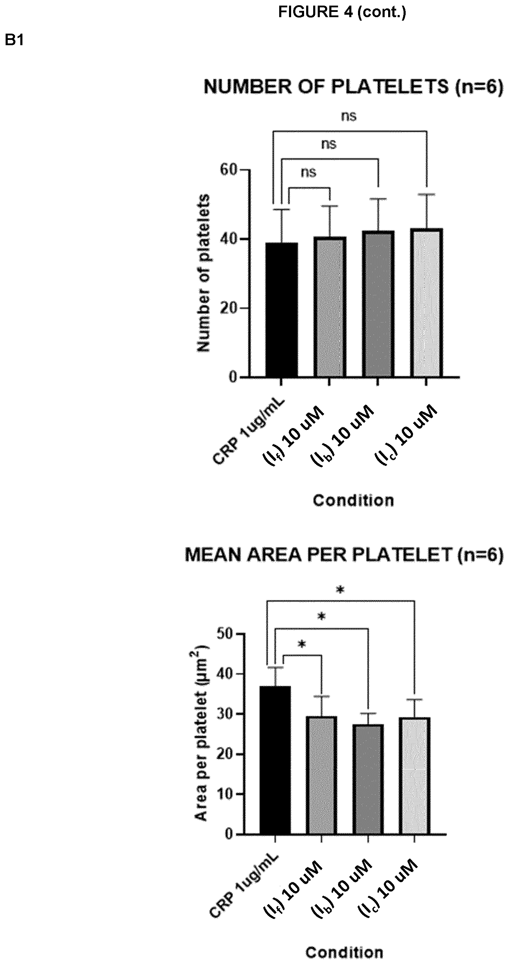

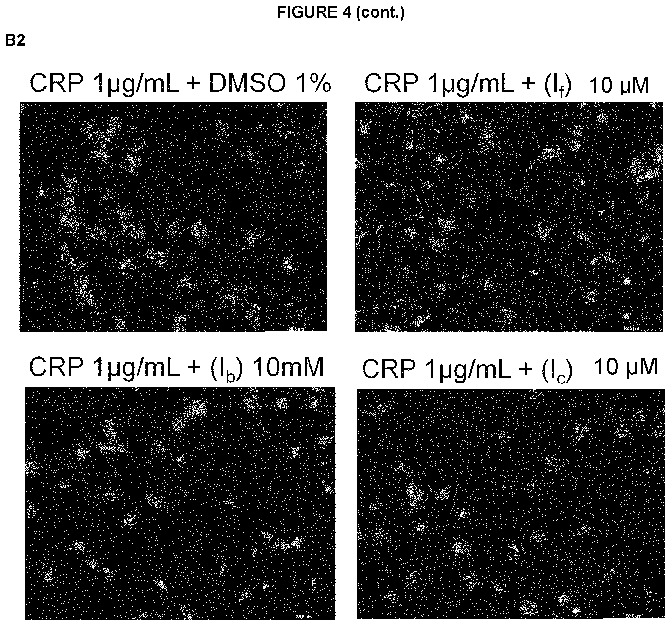

**FIG. 4****.** Platelet spreading assays. Images of platelet shape given by phalloidin staining acquired by confocal fluorescence microscopy. The inhibition of platelet spreading on CRP (1 µg/mL) is provided for compounds (I_{b}), (Ic), (I_{f}) and (Iₕ), when compared to a control where only CRP was present with 1% DMSO. Mean number of platelets and mean area per platelet were obtained when each of the test compounds (Ib), (I_{c}), (I_{f}) and (Iₕ) were administered at a 10 µM dose, respectively. **(A1)** Mean number of platelets, mean area per platelet and **(A2)** images shown for a first assay carried out with compounds (I_{f}) and (Iₕ). **(B1)** Mean number of platelets, mean area per platelet and **(B2)** images shown for a second assay carried out with compounds (I_{b}), (I_{c}) and (I_{f}).
FIG. 5. Percentage inhibition of Cytochrome P450 enzymes (A) CYPA2, (B) CYP2C19, (C) CYP2D6, (D) CYP2C9 and CYP3A4 with testosterone (E) and midazolam (F) as probes, with the compound (I_{f}) at different molar concentrations (logarithm of the molar concentration used is shown).

### EXAMPLES

The examples of the present invention described below aim to illustrate its preferred embodiments without limiting its scope of protection.

### EXAMPLE 1: Binding model of the compounds of formula (I) to GPVI

We performed a in silico study to analyze the potential interactions of the molecules of formula (I) with GPVI by using a virtual docking simulation. GPVI 2GI7 structure from PDB was used in this analysis, following a previously described protocol [Álvarez-Coiradas et al., (2020)].

Compound (Iₙ) interacts better with the target compared to compound (I_{f}): Best conformer compound (Iₕ): -9.5 kcal/mol vs best conformer compound (I_{f}): -8.1 kcal/mol.

Our preliminary observations show that no cysteine residue is involved in the interaction and binding sites for the best conformer are different, although molecules can bind to the endogenous ligands binding site (not shown).

### EXAMPLE 2: Biophysical binding assay of the compounds of formula (I) to GPVI

A biophysical assay to test direct interaction with GPVI was developed by using a label-free methodology based on dynamic mass redistribution (DMR).

This protocol consists of immobilizing the target protein in a surface by chemical means and then measure the refractory index of a light directed to the bottom of the surface, which contains a sensor. It has been used previously for other targets to determine the potency of ligands such as peptides and molecules [Akhter et al., (2017) and Álvarez-Coiradas et al., (2020)].

Briefly, EnSpire LFB high sensitivity plates (PerkinElmer 6057460, USA) were activated with 400 mM EDC (N-(3-dimethylaminopropyl)-N'- ethylcarbodiimide hydrochloride) (Sigma Aldrich, UK) and 100 mM sulfo-NHS (sulfo-N-hydroxysulfosuccinimide) (Thermo Fisher, USA) diluted in ultrapure water for 30 min at room temperature. Microplates were subsequently washed with ultrapure water. GPVI protein was immobilized (25 µg/ml) in 20 mM sodium acetate buffer, pH 5.5. After an overnight incubation at 4 °C, the microplate was wa- shed with PBS with 0.005% Tween-20, pH 7.4 (assay buffer). Baseline was read after three hours on EnSpireTM 2300 Multilabel Plate Reader (PerkinElmer, Singapore). Compounds were added to the microplate, incubated for 1 h and the final read was performed in the EnSpireTM 2300 Multilabel Plate Reader. Recombinant CRP and assay buffer were employed as a positive and a negative control, respectively.

Compounds were assayed by means of concentration-response curves with concentrations between 100 µM and 0.4 µM (1:3 serial dilutions in triplicates), while CRP was assayed similarly starting with 7µg/ml top dose and a total of 6 concentrations (1:2 serial dilutions in triplicates). Response (pm) was obtained by subtracting the baseline measurement from the final read.

We found that collagen related peptide (CRP), the natural ligand for GPVI, shows a robust dose-dependent response upon interaction with the target. Interestingly, the compound of formula (I_{f}) shows a similar target molecule interaction profile as CRP with GPVI (Figure 1).

Moreover, the compounds of formula (Iₐ), (I_{b}), (Iₑ), (I_{d}) and (Iₑ) also show (table 1 below) a similar response towards interaction at micromolar doses, which suggest binding to GPVI.

On the other hand, said compounds were evaluated versus other compounds with a similar structure, such as reference compounds A and B:

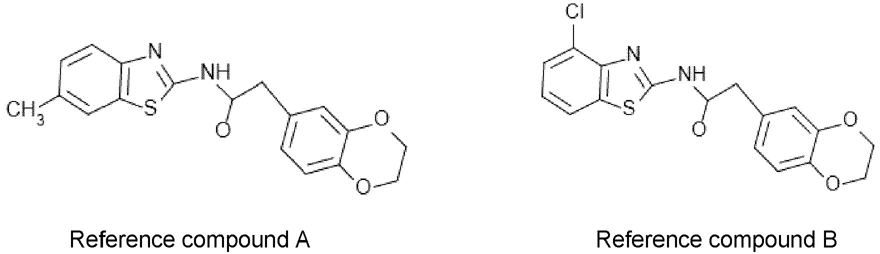

Reference compounds A and B, having a similar structure to that of the compounds of formula (I) did not show a good interaction profile (table 1 below) with GPVI, indicating that the specific characteristics of the compounds of formula (I) allow for a good interaction profile with GPVI.

**Table 1: interaction of the compounds of formula (I) with GPVI at different molar concentrations (1µM and 10µM)**

| | **Compound concentration (1µM)** | | **Compound concentration (10µM)** | |
|---|---|---|---|---|
| | **Mean** | **SD** | **Mean** | **SD** |
| Compound (Iₐ) | 10.8 | 8.08 | 19.81 | 2.89 |
| Compound (I_{b}) | 13.76 | 7.00 | 20.47 | 4.14 |
| Compound (I_{c}) | 12.37 | 5.72 | 25.42 | 3.51 |
| Compound (I_{d}) | 17.38 | 6.66 | 28.66 | 4.52 |
| Compound (Iₑ) | 6.50 | 7.63 | 25.16 | 3.35 |
| Ref. compound A | -9.78 | 3.60 | 1.91 | 12.48 |
| Ref. compound B | 6.77 | 3.78 | -16.01 | 14.48 |

### EXAMPLE 2: Effect of the compounds of formula (I) on calcium transients in human platelets.

An intracellular Ca⁺² mobilization assay was used to test the activity of the ligands in platelets. This assay measures the changes in calcium in real time by fluorophore dye Calcium 4, providing a useful way to characterize platelet activation by GPVI signaling [Barrachina et al. (2021)].

### Intracellular Ca⁺² Mobilization Assays:

Washed platelets (5x10E6) were incubated with FLIPR Calcium 4 reagent (Molecular Devices LLC, San Jose, CA, USA) in a 384-well transparent bottom plate (Greiner Bio-One International GmbH, Kremsmünster, Austria) and supplemented with Probenecid (Invitrogen^{™}, Carlsbad, CA, USA) 0.07% for 1 h at 37 °C. Platelets were co-incubated for 10 min with the tested compounds and inhibitors of secondary mediators (2 U/mL apyrase and 10 microM indomethacin (Sigma-Aldrich, Saint Louis, MO, USA)) under nonaggregating conditions (9 microM eptifibatide (Cayman Chemicals, Ann Arbor, MI, USA)). Then, the GPVI-specific agonist Collagen-Related Peptide (CRP) was added to the platelet
suspension at different doses. Both potential GPVI inhibitors and CRP were automatically dispensed by FDSS7000EX Functional Drug Screening System (Hamamatsu Photonics, Hamamatsu, Japan). Intracellular Ca²⁺ mobilization was assessed by measuring fluorescence changes (480 nm emission and 540 nm excitation wavelengths) in real time for 15 min after the addition of the agonist. 100% was expressed as maximal Ca²⁺ release in platelets treated with the agonist CRP. To determine IC₅₀, compounds were tested at 9 different concentrations in duplicates starting from 10 uM and performing 1:3 serial dilutions. The analysis of the data was performed with GraphPad Prism^{®} software (version 6.0e, March 2014) (GraphPad Software, San Diego, CA, USA) by carrying out a nonlinear regression fit for log(inhibitor) vs. relative CRP activity using a Hill slope of -1, and top and bottom constraints of 100 and 0, respectively.

The results (table 2) indicate that the compounds of formula (I) tested are capable to inhibit calcium mobilization at micromolar concentrations upon drug-induced human platelet activation with CRP:

**Table 2: Effect of the compounds of formula (I) on calcium mobilization in human platelets**

| | % Inhibition of CRP effect at 1µM | |
|---|---|---|
| | Mean | SD |
| Compound (Iₙ) | 68.16 | 1.26 |
| Compound (I_{f}) | 31.02 | 1.40 |
| Compound (Iₐ) | 53.74 | 7.20 |
| Compound (I_{b}) | 15.91 | 0.03 |
| Compound (I_{c}) | 34.05 | 43.13 |
| Compound (I_{d}) | 35.97 | 4.44 |
| Compound (Iₑ) | 24.64 | 19.30 |

### EXAMPLE 3: Solubility of the compounds of formula (I) in water

Solubility was determined by nephelometry to assess its potential impact on the bioassays at active concentrations.

Stock solutions (10⁻² M) of the assayed compounds of formula (I) were diluted to test a range of molarity from 300 µM to 0.1 µM, in 384 well transparent plate (Greiner 781801) in 1% DMSO: 99% PBS buffer solution. Then, said compounds were incubated for 2 hours at 37°C and read in a NEPHELOstar Plus (BMG LABTECH).

The results were adjusted to a segmented regression to obtain the maximum concentration in which compounds are soluble. Digoxin and Prazosin were included as reference of highly soluble drugs, while Progesterone was tested as a reference of low soluble drug, and data obtained in the experiment indicate the expected solubility profile. The active compounds show an overall solubility at concentrations above 20 µM, indicating that the bioassays can be performed in the low micromolar ranges (see table 3):

**Table 3: Solubility of compounds of formula (I). * Solubility of standards from [Dehring et al. (2004)].**

| Compound of formula (I) | Solubility (µM) | Reported solubility (µM) |
|---|---|---|
| (I_{f}) | 61.5 | |
| (Iₐ) | 5.4 | |
| (I_{b}) | 50.0 | |
| (I_{c}) | 51.9 | |
| (I_{d}) | 21.9 | |
| (Iₑ) | 22.5 | |
| Digoxin | 96.9 | 59.3* |
| Prazosin | 77.2 | 26.3* |
| Progesterone | 5.1 | 3.7* |

### EXAMPLE 4: Effect of the compounds of formula (I) in aggregation of human platelets

Platelet aggregation assays were carried out to assess the potency of the active compounds as previously described [Barrachina et al. (2021)].

Briefly, human platelets from at least =7 healthy individuals were isolated and washed. Then, the suspension was incubated with the compounds at 10 µM at 37 ∘C for 4 min in a Chrono-log^{®} 490-X aggregometer (Chrono-log Corporation, Havertown, PA, USA). After that, platelets were stimulated with the agonist CRP at 1 µg/mL for 5 min.

The data shows consistent inhibition of platelet aggregation for the compounds of formula (I): (I_{b}), (I_{c})and (Iₑ) (Figure 2A, 2B and 2C respectively), although with lower potency than compound (Iₙ) and compound (I_{f})(56 ±20% inhibition for compound (Iₕ), and 70±19% for compound (I_{f})).

In addition, an assessment of the selectivity of the active compound (I_{c})at 10 µM was carried out based on aggregation assays. Briefly, human platelets from healthy individuals were isolated and washed. The agonists used in this selectivity assay were collagen, rhodocytin, and thrombin.

The data shows consistent inhibition of platelet aggregation by compound (I_{c}) in platelets exposed to collagen (Figure 3A), while aggregation induced by rhodocytin, and thrombin remain unchanged (Figures 3B and 3C respectively).

### EXAMPLE 5: Effect of the compounds of formula (I) in spreading of human platelets

Platelet spreading experiments were carried out to assess the potency of the compounds of formula (I), exemplified by compounds (I_{f})and (Iₕ).

Platelets can change from a rounded, non-adherent form to adhere and flatten shape when exposed to ligands. This "spreading" effect can lead to the formation of cytoskeletal protein rearrangement. Platelet adhesion and extent of spreading (area covered) can be quantified.

Briefly, human platelets from healthy individuals were isolated and washed. Then, platelets were incubated with the inhibitors at 10µM for 5 min at room temperature and then for 45 min at 37°C in coverslips coated with 1µg/mL of agonist (CRP).

The images of platelet shape given by phalloidin staining were acquired by confocal fluorescence microscopy and the area analyzed by ImageJ [Onselaer et al., (2020)]. The data shows inhibition of platelet spreading on CRP for compounds (I_{b}), (I_{c}), (I_{f})and (Iₕ), consistent with the inhibitory effect on aggregation (see mean number and mean area of platelets).

### EXAMPLE 6: Effect of the compounds of formula (I) in the viability of human platelets.

The potential toxicity of the compounds of formula (I) was also evaluated. The assay uses calcein-AM as a marker of platelet viability. Calcein-AM is a membrane permeable nonfluorescent molecule, which is converted to fluorescent calcein by intracellular esterases.

### Viability-Related Calcein-AM Flow Cytometry Assay

To measure the potential toxicity of inhibitors, a viability assay based on Calcein-AMbinding measured by flow cytometry was performed. Washed platelets (2.5x10E8 platelets/mL) were incubated for 10 min with 10 uM of the compounds to be tested or 0.1% DMSO (Sigma-Aldrich, Saint Louis, MO, USA) as vehicle. Then, 1 uL of Calcein-AM 0.1 mg/mL (Sigma-Aldrich, Saint Louis, MO, USA) was added to the suspension and incubated for an additional 20 min at room temperature. Calcein-AM binding was measured in triplicate by an Accuri C6 flow cytometer (BD Biosciences, San Jose, CA, USA). Data were analyzed comparing the average of the percentage of positive events in the presence of the inhibitor with the average of the percentage of positive events in the absence of the inhibitor (vehicle

Overall, the compounds of formula (I) evaluated at 5 and 10 µM do not affect significantly viability.

### EXAMPLE 7: Plasmatic and metabolic stability, plasma protein binding and CYP inhibition of the compounds of formula (I)

### 1.- PLASMATIC STABILITY

Rat, human, mouse and dog plasma pooled from healthy donors extracted in citrate tubes was employed in the assay.

Plates containing 5µM compounds in plasma (total volume: 100µL) were incubated at 37°C at the different times (0, 60, 120 and 360 min). Then 300µl Acetonitrile were added for precipitating plasma protein, and the plate was centrifuged at 4000 g for 60 min at 4°C.

Supernatant was taken and analyzed by UPLC/MS/MS for sample quantification.
Stationary phase: Reverse phase ACQUITY BEH C18 1.7µm 2.1x50mm (Waters)
Mobile phase: 0.1% Formic acid water/0.1% formic acid in acetonitrile
Gradient:

**Table 4: UPLC gradient / Plasmatic stability assay**

| **Time** | **Water** | **Acetonitrile** |
|---|---|---|
| 0.0 | 95% | 5% |
| 0.1 | 95% | 5% |
| 1 | 0% | 100% |
| 2 | 0% | 100% |
| 2.1 | 95% | 5% |
| 2.5 | 95% | 5% |

Flow: 0.6 ml/min.

The chromatographic equipment employed was an UPLC QSM Waters Acquity. Compound concentrations were calculated from the MS peak areas.

### Results:

**Table 5: Plasmatic stability of the compounds of formula (I).**

| | | t(min) | | | |
|---|---|---|---|---|---|
| | | 0 | 60 | 120 | 360 |
| (I_{f}) 10µM | Rat | 100.0 | 105.5 | 105.4 | 81.6 |
| | Human | 100.0 | 101.0 | 101.7 | 101.4 |
| | Mouse | 100.0 | 67.8 | 41.6 | 5.6 |
| | Dog | 100.0 | 89.2 | 85.1 | 83.5 |
| (I_{f}) **100µM** | Rat | 100.0 | 88.8 | 95.1 | 65.7 |
| | Human | 100.0 | 104.3 | 107.4 | 106.4 |
| | Mouse | 100.0 | 77.6 | 57.5 | 9.6 |
| | Dog | 100.0 | 101.4 | 106.5 | 102.4 |

As seen from the results shown in table 5, the compound of formula (If) has a high plasmatic stability in human (maximum levels) and rats, but not on mice.

### 2.-METABOLIC STABILITY

Microsomes from Tebu-bio Xenotech were employed in the assay, having a content of 20 mg/ml of protein. The following quantities were added to each well of a 96-well microplate.

**Table 6: Metabolic stability assay.**

| | | **Rat (µl)** | **Mouse (µl)** | **Dog (µL)** | **Human (µl)** |
|---|---|---|---|---|---|
| **Phosphate buffer Na/K 50 mM pH 7.4** | | 301 | 310 | 308 | 296 |
| **MgCl2 30 mM** | COFACTORS | 163 | 163 | 163 | 162.5 |
| **NADP 10 mM** | | | | | |
| **Glucose 6-P 100mM** | | | | | |
| **Glucose 6-P DH 40 U/ml** | | | | | |
| **Rat microsomes** | | 31.4 | | | |
| **Human microsomes** | | | | | 36.5 |
| **Mouse microsomes** | | | 22.4 | | |
| **Dog microsomes** | | | | 24.5 | |
| **Test compound** | | 5 | 5 | 5 | 5 |
| **Total volume (µL)** | | 500 | 500 | 500 | 500 |

Plates were incubated at 37°C and 75 µL samples were taken at 0, 10, 20, 40 and 60 minutes.

Samples were transferred to a microplate and 75 µl Acetonitrile + IS(Rolipram) were added for inactivating the microsomes, and 30 µl of H₂O with 0.5% formic acid for improving the chromatographic conditions and kept at 4°C. When all the samples were taken the plate was centrifuged at 46000 g for 30 min at 15°C.
Stationary phase: ACQUITY BEH C18 1.7µm 2.1x50mm (Waters)
Mobile phase: A: 0.1% formic; B: acetonitrile+0.1% formic acid Gradient:

**Table 6: UPLC gradient / Metabolic stability assay**

| **Time (min)** | **A** | **B** |
|---|---|---|
| 0.0 | 95% | 5% |
| 0.1 | 95% | 5% |
| 1.0 | 0% | 100% |
| 2.0 | 0% | 100% |
| 2.1 | 95% | 5% |
| 2.5 | 95% | 5% |

Flow: 0.6 ml/min

The chromatographic equipment employed was an UPLC QSM Waters Acquity.

Metabolic stability was calculated from the logarithm of the remaining compound at each of the times evaluated.

| Compound | Rat | | | Mouse | | |
|---|---|---|---|---|---|---|
| | % remanent (*) | t1/2 (min) | Clint (µL/min*mg prot) | % remanent (*) | t1/2 (min) | Clint (µL/min*mg prot) |
| (I_{f}) | 49.9 | 62.1 | 8.89 | 71.6 | 109 | 7.09 |
| Testosterone | 0.14 | 0.9 | 610 | 0.06 | 4.76 | 163 |

**Table 7: Metabolic stability of the compounds of formula (I) in rat, mice, dogs and humans / (*) sampling time 60 min.**

| Compound | Dog | | | Human | | |
|---|---|---|---|---|---|---|
| | % remanent (*) | t1/2 (min) | Clint (µL/min*mg prot) | % remanent (*) | t1/2 (min) | Clint (µL/min*mg prot) |
| (I_{f}) | 89.2 | 325 | 2.17 | 41.21 | 46.88 | 10.13 |
| Testosterone | 49.1 | 59.9 | 11.8 | 4.02 | 12.95 | 36.66 |

### 3.- PLASMA PROTEIN BINDING:

Plasma from Seralab was employed in the assay and was carried out by employing Rapid Equilibrium Dialysis (RED) from Thermo Scientific.

The compounds were dissolved at 5 µM in plasma and added to the corresponding insert of the RED device. Dialysis buffer was added to the corresponding insert of the RED device. Plate was incubated for 4h at 37°C.

After the incubation period 50 µL aliquots of each chamber were transferred to empty vials. 50 µL of dialysis buffer were added to the plasma samples and 50 µL of plasma were added to the buffer samples. 300 µL of acetonitrile were added to all the samples and centrifuged at 4000 rpm 100 µL aliquots of the supernatants were transferred to a LC analysis plate.
Samples were analyzed in a UPLC/MS/MS.
Stationary phase: Acquity UPLC^{®} BEHC18 1,7 µm (2.1 mm x 50 mm) (Waters).
Gradient: A (H₂O + 0.1% Formic acid); B (ACN + 0.1% Formic acid);
Flow: 0.6mL/min

**Table 8: UPLC gradient / Protein binding assay**

| **Time (min)** | **A** | **B** |
|---|---|---|
| 0.0 | 95% | 5% |
| 0.1 | 95% | 5% |
| 1.0 | 0% | 100% |
| 2.0 | 0% | 100% |
| 2.1 | 95% | 5% |
| 2.5 | 95% | 5% |

The chromatographic equipment employed was an ACQUITY UPLC / Xevo TQD System. Compound concentrations were calculated from the MS peak areas.

**Table 9: Plasma protein binding of the compounds of formula (I) in rat, mice, dogs and humans / (*) low recovery**

| Compound | | Plasma protein binding (%) | Unbound fraction (%) | Acceptance range PPB Phenytoin (%) |
|---|---|---|---|---|
| (I_{f}) | RAT | 99.90 | 0.10 | |
| | HUMAN | 99.89 | 0.11 | |
| | MOUSE* | 99.54 | 0.46 | |
| | DOG | 99.85 | 0.15 | |
| Phenytoin | RAT | 86.24 | 13.76 | 73,0-89,0 |
| | HUMAN | 82.23 | 17.77 | 78,4-94,4 |
| | MOUSE | 82.06 | 17.94 | 73,5-89,5 |
| | DOG | 81.06 | 18.94 | 70,4-86,4 |

As seen in table 9, the compound (If) showed very high plasma protein binding.

### 4.- CYP INHIBITION:

Cytochrome P450 (CYP) are a family of enzymes which play a major role in the metabolism of drugs. Assessment of the potential of a compound to inhibit a specific cytochrome P450 enzyme is important as co-administration of compounds may result in one or both inhibiting the other's metabolism.

All the incubations were performed in 250 µL reaction medium containing sodium/potassium phosphate buffer 50mM, pH 7.4, 3mM MgCl2, an NADPH regeneration system 1mM NADP, 10mM glucose 6-phosphate, and 1 U/mL glucose 6-phosphate dehydrogenase, 0.5 mg protein/mL of liver microsomes (HLM Xenotech), and a substrate cocktail composed of phenacetin for CYP1A2, diclofenac for CYP2C9, S-mephenytoin for CYP2C19, bufuralol for CYP2D6, midazolam for CYP3A4, and testosterone for CYP3A4. Final concentrations of substrate in the reaction medium were: 10 µM phenacetin, 10 µM diclofenac, 5 µM bufuralol, 100µM S-Mephenytoin, 5 µM midazolam, and 50 µM testosterone. Following incubation at 37°C for 25 min, the reactions were terminated by the addition of ice-cold acetonitrile and the plate was centrifuged at 4000 g for 60 min at 4°C.

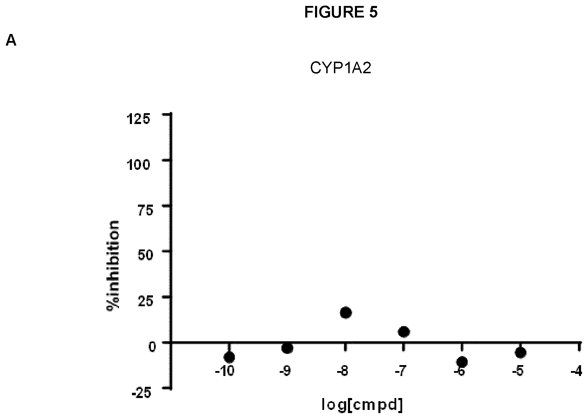

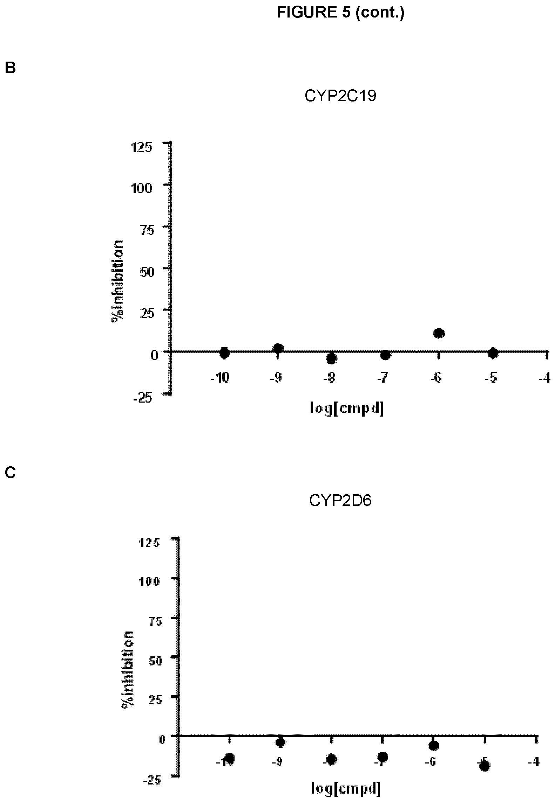

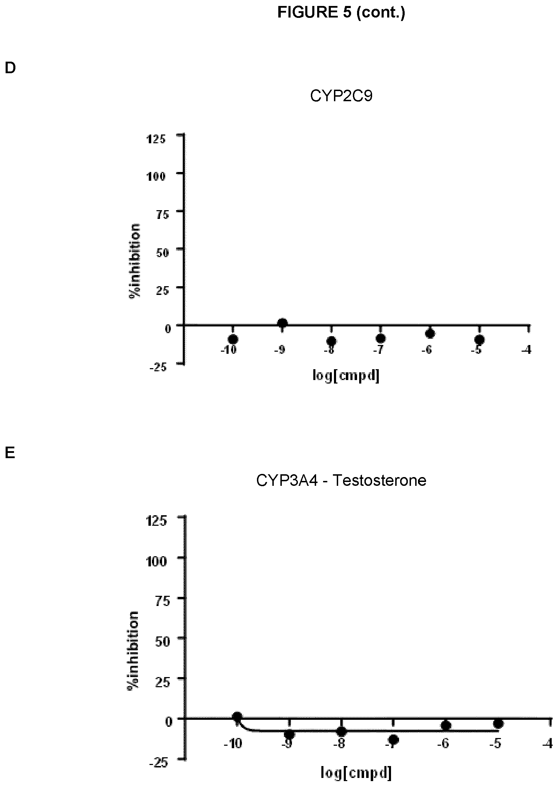

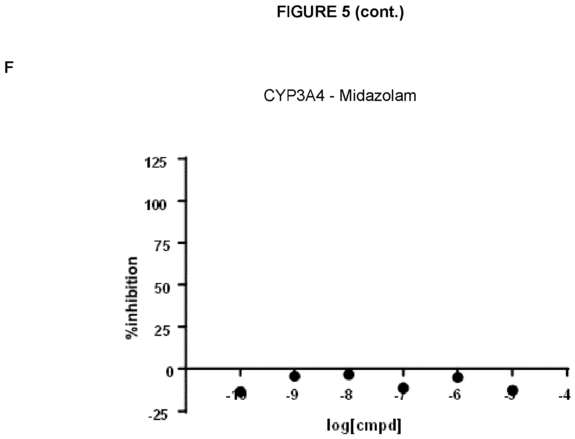

The compound of formula (I) used in the inhibition assay was compound (I_{f}) at different concentrations (figure 5 shows logarithm of the different molar concentrations used vs % of inhibition of CYP enzyme).

Supernatant was taken and analyzed by UPLC/MS/MS for sample quantification The chromatographic equipment employed was an UPLC QSM Waters Acquity.
Stationary phase: Reverse phase Acquity UPLC^{®} BEHC18 1,7 µm (2.1 mm x 100 mm) (Waters)
Mobile phase: 0.1% Formic acid water/0.1% formic acid in acetonitrile. Flow: 0.8 ml/min. Gradient:

**Table 10: UPLC gradient / CYP inhibition assay**

| **Time** | **Water** | **Acetonitrile** |
|---|---|---|
| 0 | 100% | 0% |
| 0.1 | 100% | 0% |
| 0.5 | 2% | 98% |
| 0.75 | 2% | 98% |
| 1 | 100% | 0% |

The results for compound (I_{f}) are shown in table 11, below, as well as in figures 5A-F.

**Table 11: CYP inhibition assay with the compounds of formula (I)**

| | % inhibition (10µM) | IC₅₀ (µM) |
|---|---|---|
| CYP1A2 | -5.3 | >10 |
| CYP2C9 | -0.7 | >10 |
| CYP2C19 | -18.8 | >10 |
| CYP2D6 | -2.9 | >10 |
| CYP3A4 (Midazolam) | -9.4 | >10 |
| CYP3A4 (Testosterone) | -12.8 | >10 |

### REFERENCES

Alvarez-Coiradas E, Munteanu CR, Diaz-Saez L, Pazos A, Huber KVM, Loza MI, et al. Discovery of novel immunopharmacological ligands targeting the IL-17 inflammatory pathway. Int Immunopharmacol. 2020 Dec;89(Pt A):107026.
Akhter S, Chakraborty S, Moutinho D, et al. The human VGF-derived bioactive peptide TLQP-21 binds heat shock 71 kDa protein 8 (HSPA8) on the surface of SH-SY5Y cells. PLoS One. 2017 Sep 21;12(9):e0185176.
Ashorobi D, Ameer MA, Fernandez R. Thrombosis. [Updated 2021 Dec 29]. In: StatPearls [Internet]. Treasure Island (FL): StatPearls Publishing; 2022
Barrachina N, Izquierdo I, Hermida-Nogueira L, et al. The PI3Kδ Inhibitor Idelalisib Diminishes Platelet Function and Shows Antithrombotic Potential. Int J Mol Sci. 2021 Mar 24;22(7):3304.
Dehring KA, Workman HL, Miller KD, Mandagere A, Poole SK. Automated robotic liquid handling/laser-based nephelometry system for high throughput measurement of kinetic aqueous solubility. J Pharm Biomed Anal. 2004 Nov 15;36(3):447-56
Lockyer S, Okuyama K, Begum S, Le S, Sun B, Watanabe T, Matsumoto Y, Yoshitake M, Kambayashi J, Tandon NN. GPVI-deficient mice lack collagen responses and are protected against experimentally induced pulmonary thromboembolism. Thromb Res. 2006;118(3):371-80.
Onselaer M-B, Nagy M, Pallini C, Pike JA, Perrella G, Quintanilla LG, et al. Comparison of the GPVI inhibitors losartan and honokiol. Platelets. 2020;31(2):187-97.

## Claims

1. A compound of formula (I), or a pharmaceutically acceptable salt or solvate thereof: 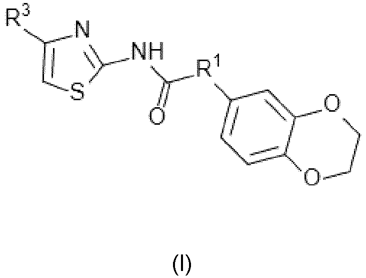
wherein R¹ is selected from a group consisting of -CH₂- or a group R²: 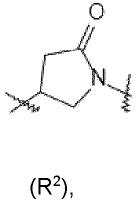
wherein R² is attached to the 2,3-dihydrobenzo[b][1,4]dioxine by the nitrogen atom of the pyrrolidin-2-one ring; wherein R³ is selected from the group consisting of a phenyl substituted with one or more groups R⁴, a naphthyl group substituted with one or more groups R⁴, a 1,2,3,4-tetrahydronapht-6-yl group substituted with one or more groups R⁴ and a 2-oxo-1,2,3,4-tetrahydroquinolin-6-yl group; and wherein R⁴ is H or a halogen;
for use as a GPVI inhibitor in the prevention and/or treatment of thrombosis, as well as pathologies associated to thrombus formation selected from: ischemic stroke, atherosclerosis, coronary artery disease or myocardial infarction.

2. The compound of formula (I) for use according to claim 1 selected from the group consisting of Iₐ, I_{b}, I_{c}, I_{d}, Iₑ, I_{f} and Iₕ:

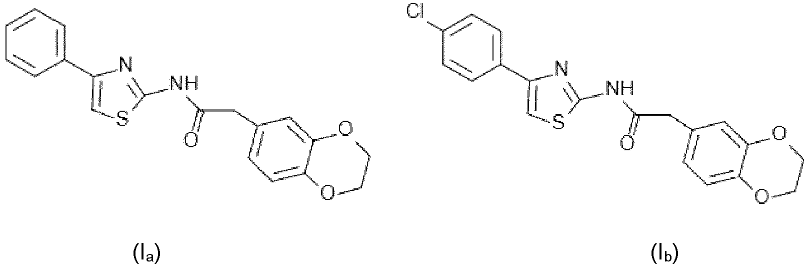

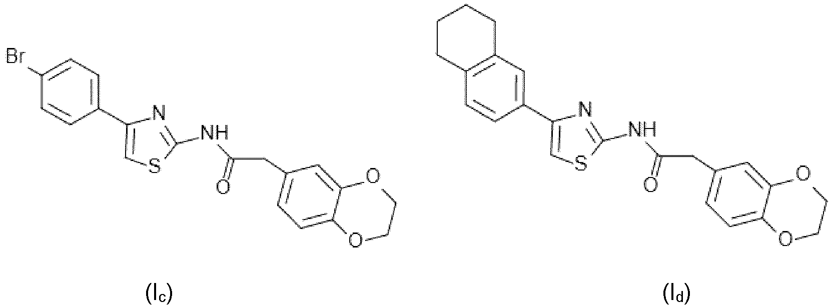

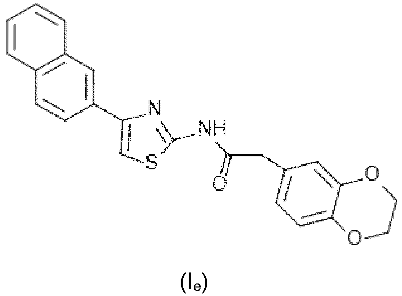

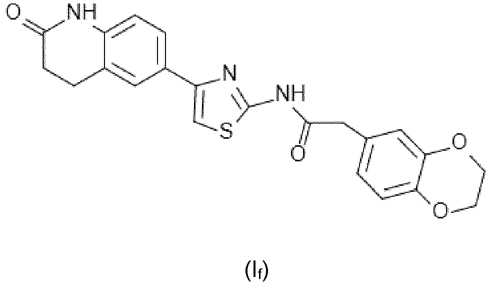

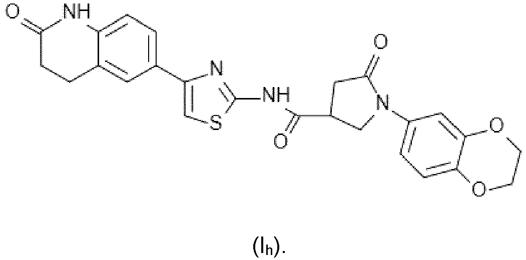

3. The compound of formula (I) for use according to any of claims 1 or 2, wherein said compound of formula (I) is administered prior to, in combination with, or subsequently after administering an additional therapeutic agent for use in the prevention and/or treatment of thrombosis, as well as pathologies associated to thrombus formation selected from: ischemic stroke, atherosclerosis, coronary artery disease or myocardial infarction.

4. The compound of formula (I) for use according to claim 3, wherein said additional therapeutic agent is an antiplatelet drug.

5. The compound of formula (I) for use according to any of claims 3 or 4, wherein said additional therapeutic agent is selected from the group consisting of a cyclooxygenase inhibitor, an adenosine diphosphate receptor inhibitor, a phosphodiesterase inhibitor, protease-activated receptor-1 antagonist, a glycoprotein IIB/IIIA inhibitor, an adenosine reuptake inhibitor and a thromboxane inhibitor.

6. A pharmaceutical composition comprising at least one compound of formula (I), or a pharmaceutically acceptable salt or solvate thereof: 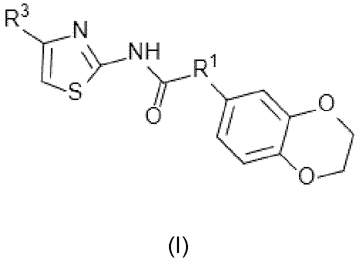
wherein R¹ is selected from a group consisting of -CH₂- or a group R²: 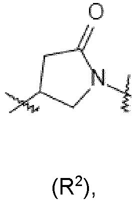
wherein R² is attached to the 2,3-dihydrobenzo[b][1,4]dioxine by the nitrogen atom of the pyrrolidin-2-one ring; wherein R³ is selected from the group consisting of a phenyl substituted with one or more groups R⁴, a naphthyl group substituted with one or more groups R⁴, a 1,2,3,4-tetrahydronapht-6-yl group substituted with one or more groups R⁴ and a 2-oxo-1,2,3,4-tetrahydroquinolin-6-yl group; and wherein R⁴ is H or a halogen;
and at least one pharmaceutically acceptable excipient, for use as a GPVI inhibitor in the prevention and/or treatment of thrombosis, as well as pathologies associated to thrombus formation selected from: ischemic stroke, atherosclerosis, coronary artery disease or myocardial infarction.

7. The pharmaceutical composition for use according to claim 6, wherein said compound of formula (I) is selected from the group consisting of Iₐ, I_{b}, I_{c}, I_{d}, Iₑ, I_{f} and Iₕ:

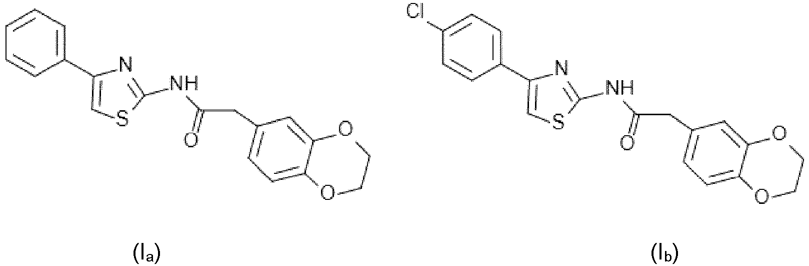

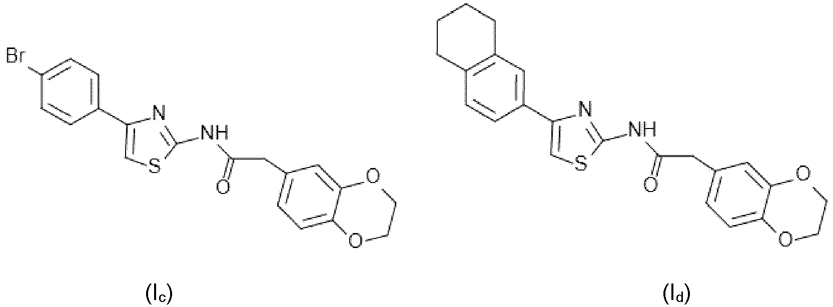

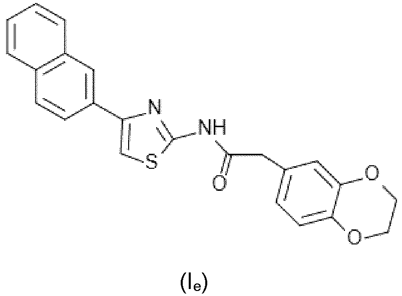

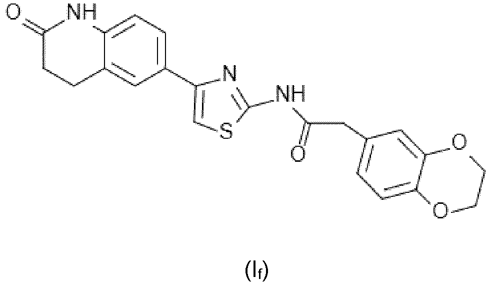

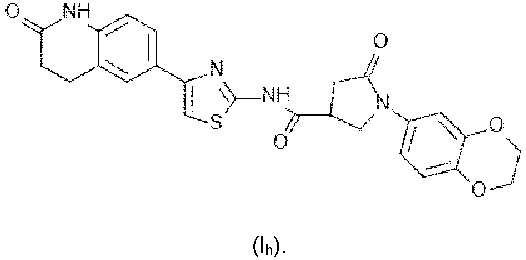

8. The pharmaceutical composition for use according to any of claims 6 or 7, wherein said pharmaceutical composition is administered prior to, together with, or after administering an additional therapeutic agent for use in the prevention and/or treatment of thrombosis.

9. The pharmaceutical composition for use according to claim 8, wherein said pharmaceutical composition further comprises another additional therapeutic agent for use in the prevention and/or treatment of thrombosis.

10. The pharmaceutical composition for use according to any of claims 8 or 9, wherein said additional therapeutic agent is an antiplatelet drug.

11. The pharmaceutical composition for use according to any of claims 8 to 10, wherein said additional therapeutic agent is selected from the group consisting of a cyclooxygenase inhibitor, an adenosine diphosphate receptor inhibitor, a phosphodiesterase inhibitor, protease-activated receptor-1 antagonist, a glycoprotein IIB/IIIA inhibitor, an adenosine reuptake inhibitor and a thromboxane inhibitor.

12. The pharmaceutical composition for use according to any of claims 6 to 11, wherein said pharmaceutical composition is administered orally, transdermally or parenterally.

13. Use of a compound of formula (I) in an *in-vitro* test system for detection of GPVI-mediated platelet activation.

## Patentansprüche

1. Eine Verbindung der Formel (I) oder ein pharmazeutisch verträgliches Salz oder Solvat davon: 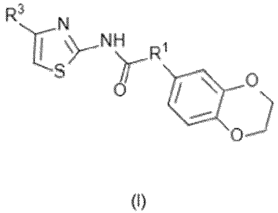
wobei R¹ aus einer Gruppe ausgewählt ist, bestehend aus -CH₂- oder einer Gruppe R²: 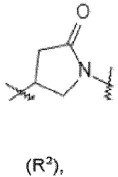
wobei R² durch das Stickstoffatom des Pyrrolidin-2-on-Rings an das 2,3-Dihydrobenzo[b][1,4]dioxin gebunden ist; wobei R³ aus der Gruppe ausgewählt ist, bestehend aus einem Phenyl, das mit einer oder mehreren Gruppen R⁴ substituiert ist, einer Naphthylgruppe, die mit einer oder mehreren Gruppen R⁴ substituiert ist, einer 1,2,3,4-Tetrahydronapht-6-yl-Gruppe, die mit einer oder mehreren Gruppen R⁴ substituiert ist, und einer 2-Oxo-1,2,3,4-Tetrahydrochinolin-6-yl-Gruppe; und wobei R⁴ H oder ein Halogen ist;
zur Verwendung als GPVI-Inhibitor bei der Vorbeugung und/oder Behandlung von Thrombose und Pathologien, die mit Thrombosebildung assoziiert sind ausgewählt aus: ischämischem Schlaganfall, Atherosklerose koronarer Herzkrankheit oder Myokardinfarkt.

2. Die Verbindung der Formel (I) zur Verwendung nach Anspruch 1, ausgewählt aus der Gruppe, bestehend aus Iₐ, I_{b}, I_{c}, I_{d}, Iₑ, I_{f} und Iₕ:

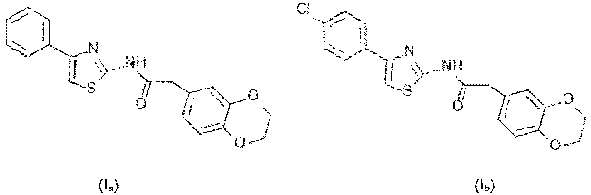

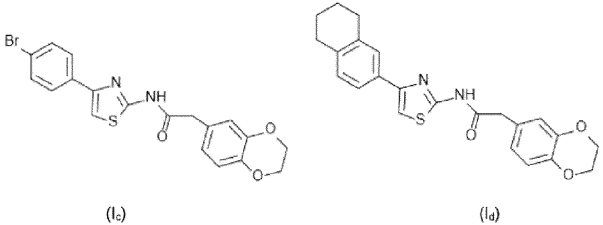

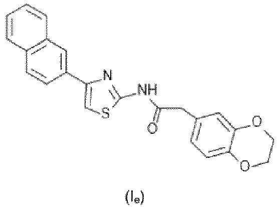

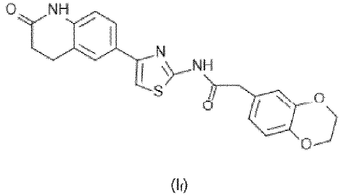

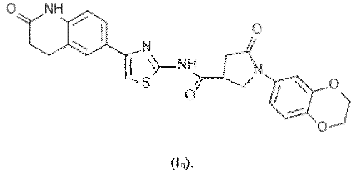

3. Die Verbindung der Formel (I) zur Verwendung nach einem der Ansprüche 1 oder 2, wobei die Verbindung der Formel (I) vor, in Kombination mit oder anschließend nach der Verabreichung eines zusätzlichen therapeutischen Mittels zur Verwendung bei der Vorbeugung und/oder Behandlung von Thrombose und Pathologien, die mit Thrombosebildung assoziiert sind , ausgewählt aus: ischämischem Schlaganfall, Atherosklerose, koronarer Herzkrankheit oder Myokardinfarkt.

4. Die Verbindung der Formel (I) zur Verwendung nach Anspruch 3, wobei das zusätzliche therapeutische Mittel ein Thrombozytenaggregationshemmer ist.

5. Die Verbindung der Formel (I) zur Verwendung nach einem der Ansprüche 3 oder 4, wobei das zusätzliche therapeutische Mittel aus der Gruppe ausgewählt ist, bestehend aus einem Cyclooxygenase-Inhibitor, einem Adenosindiphosphat-Rezeptor-Inhibitor, einem Phosphodiesterase-Inhibitor, einem Protease-aktivierten Rezeptor-1-Antagonisten, einem Glykoprotein-IIB/IIIA-Inhibitor, einem Adenosin-Wiederaufnahme-Inhibitor und einem Thromboxan-Inhibitor.

6. Eine pharmazeutische Zusammensetzung, die mindestens eine Verbindung der Formel (I) oder ein pharmazeutisch verträgliches Salz oder Solvat davon umfasst: 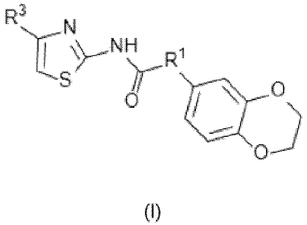
wobei R¹ aus einer Gruppe ausgewählt ist, bestehend aus -CH₂- oder einer Gruppe R²: 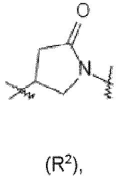
wobei R² durch das Stickstoffatom des Pyrrolidin-2-on-Rings an das 2,3-Dihydrobenzo[b][1,4]dioxin gebunden ist; wobei R³ aus der Gruppe ausgewählt ist, bestehend aus einem Phenyl, das mit einer oder mehreren Gruppen R⁴ substituiert ist, einer Naphthylgruppe, die mit einer oder mehreren Gruppen R⁴ substituiert ist, einer 1,2,3,4-Tetrahydronapht-6-yl-Gruppe, die mit einer oder mehreren Gruppen R⁴ substituiert ist, und einer 2-Oxo-1,2,3,4-Tetrahydrochinolin-6-yl-Gruppe; und wobei R⁴ H oder ein Halogen ist;
und mindestens einen pharmazeutisch verträglichen Hilfsstoff zur Verwendung als GPVI-Inhibitor bei der Vorbeugung und/oder Behandlung von Thrombose und Pathologien, die mit Thrombusbildung assoziiert sind, ausgewählt aus: ischämischem Schlaganfall, Atherosklerose, koronarer Herzkrankheit oder Myokardinfarkt.

7. Die pharmazeutische Zusammensetzung zur Verwendung nach Anspruch 6, wobei die Verbindung der Formel (I) aus der Gruppe ausgewählt ist, bestehend aus Iₐ, I_{b}, I_{c}, I_{d}, Iₑ, I_{f} und Iₕ:

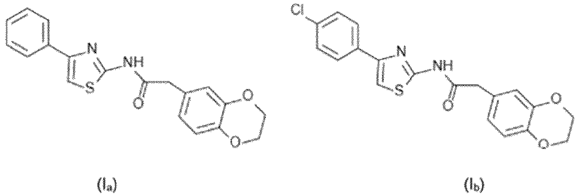

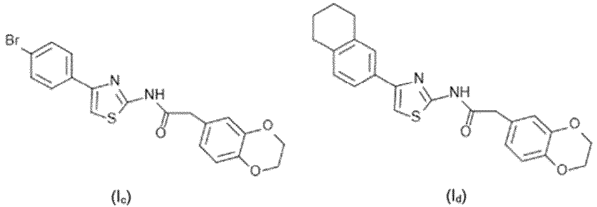

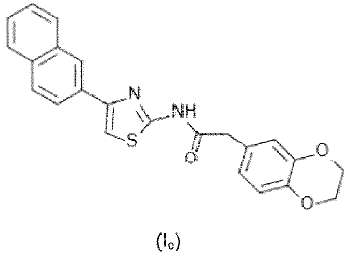

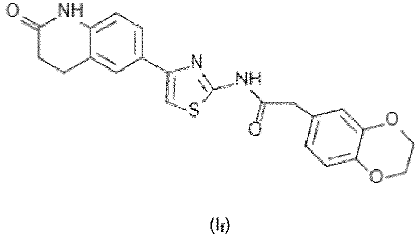

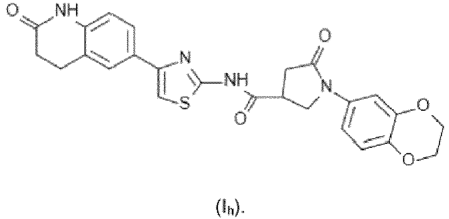

8. Die pharmazeutische Zusammensetzung zur Verwendung nach einem der Ansprüche 6 oder 7, wobei die pharmazeutische Zusammensetzung vor, zusammen mit oder nach der Verabreichung eines zusätzlichen therapeutischen Mittels zur Verwendung bei der Vorbeugung und/oder Behandlung von Thrombose verabreicht wird.

9. Die pharmazeutische Zusammensetzung zur Verwendung nach Anspruch 8, wobei die pharmazeutische Zusammensetzung ferner ein weiteres zusätzliches therapeutisches Mittel zur Verwendung bei der Vorbeugung und/oder Behandlung von Thrombose umfasst.

10. Die pharmazeutische Zusammensetzung zur Verwendung nach einem der Ansprüche 8 oder 9, wobei das zusätzliche therapeutische Mittel ein Thrombozytenaggregationshemmer ist.

11. Die pharmazeutische Zusammensetzung zur Verwendung nach einem der Ansprüche 8 bis 10, wobei das zusätzliche therapeutische Mittel aus der Gruppe ausgewählt ist, bestehend aus einem Cyclooxygenase-Inhibitor, einem Adenosindiphosphat-Rezeptor-Inhibitor, einem Phosphodiesterase-Inhibitor, einem Protease-aktivierten Rezeptor-1-Antagonisten, einem Glykoprotein-IIB/IIIA-Inhibitor, einem Adenosin-Wiederaufnahme-Inhibitor und einem Thromboxan-Inhibitor.

12. Die pharmazeutische Zusammensetzung zur Verwendung nach einem der Ansprüche 6 bis 11, wobei die pharmazeutische Zusammensetzung oral, transdermal oder parenteral verabreicht wird.

13. Verwendung einer Verbindung der Formel (I) in einem in-vitro-Testsystem zum Nachweis einer GPVIvermittelten Thrombozytenaktivierung.

## Revendications

1. Composé de formule (I), ou sel ou solvate pharmaceutiquement acceptable de celui-ci : 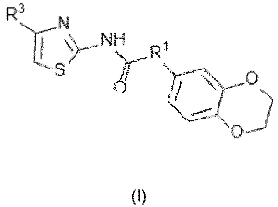
dans laquelle R¹ est choisi dans un groupe constitué de -CH₂- ou un groupe R² : 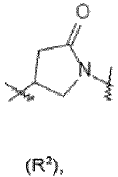
dans laquelle R² est attaché à la 2,3-dihydrobenzo[b][1,4]dioxine par l'atome d'azote du cycle pyrrolidin-2-one ; dans laquelle R³ est choisi dans le groupe constitué d'un phényle substitué par un ou plusieurs groupes R⁴, d'un groupe naphtyle substitué par un ou plusieurs groupes R⁴, d'un groupe 1,2,3,4-tétrahydronapht-6-yle substitué par un ou plusieurs groupes R⁴ et d'un groupe 2-oxo-1,2,3,4-tétrahydroquinolin-6-yle ; et dans laquelle R⁴ est H ou un halogène ;
pour une utilisation comme inhibiteur de GPVI dans la prévention et/ou le traitement de la thrombose, ainsi que des pathologies associées à la formation d'un thrombus choisies parmi : accident ischémique cérébral, athérosclérose, maladie coronarienne ou infarctus du myocarde.

2. Composé de formule (I) pour une utilisation selon la revendication 1 choisi dans le groupe constitué de Iₐ, I_{b}, I_{c}, I_{d}, Iₑ, I_{f} et Iₕ:

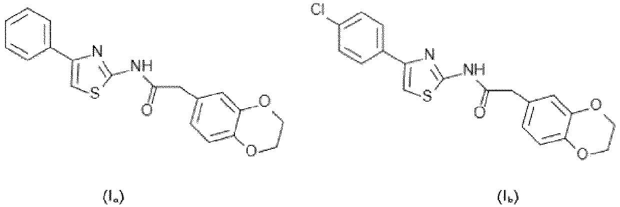

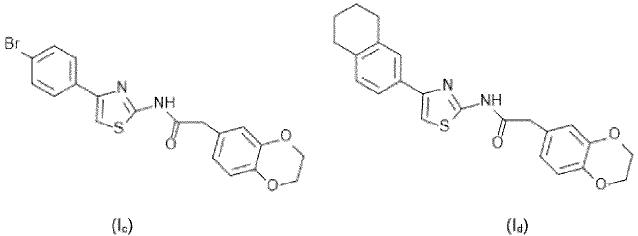

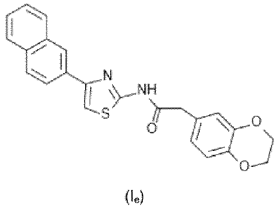

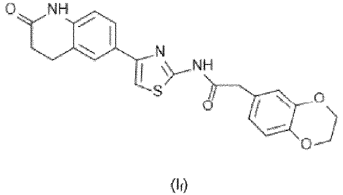

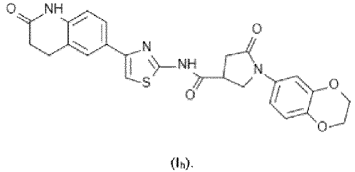

3. Composé de formule (I) pour une utilisation selon l'une quelconque des revendications 1 ou 2, ledit composé de formule (I) étant administré avant, en combinaison avec ou après l'administration d'un agent thérapeutique supplémentaire pour une utilisation dans la prévention et/ou le traitement de la thrombose, ainsi que des pathologies associées à la formation d'un thrombus choisies parmi : accident ischémique cérébral, athérosclérose, maladie coronarienne ou infarctus du myocarde.

4. Composé de formule (I) pour une utilisation selon la revendication 3, dans lequel ledit agent thérapeutique supplémentaire est un médicament antiplaquettaire.

5. Composé de formule (I) pour une utilisation selon l'une quelconque des revendications 3 ou 4, dans lequel ledit agent thérapeutique supplémentaire est choisi dans le groupe constitué d'un inhibiteur de la cyclooxygénase, d'un inhibiteur du récepteur de l'adénosine diphosphate, d'un inhibiteur de la phosphodiestérase, d'un antagoniste du récepteur-1 activé par la protéase, d'un inhibiteur de la glycoprotéine IIB/IIIA, d'un inhibiteur de la recapture de l'adénosine et d'un inhibiteur de la thromboxane.

6. Composition pharmaceutique comprenant au moins un composé de formule (I), ou un sel ou un solvate pharmaceutiquement acceptable de celui-ci : 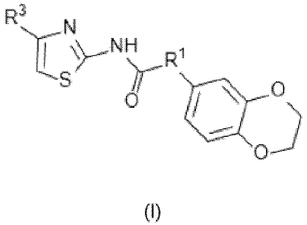
dans laquelle R¹ est choisi dans un groupe constitué de-CH₂- ou un groupe R² : 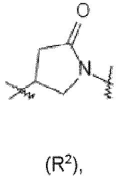
dans laquelle R² est attaché à la 2,3-dihydrobenzo[b][1,4]dioxine par l'atome d'azote du cycle pyrrolidin-2-one ; dans laquelle R³ est choisi dans le groupe constitué d'un phényle substitué par un ou plusieurs groupes R⁴, d'un groupe naphtyle substitué par un ou plusieurs groupes R⁴, d'un groupe 1,2,3,4-tétrahydronapht-6-yle substitué par un ou plusieurs groupes R⁴ et d'un groupe 2-oxo-1,2,3,4-tétrahydroquinolin-6-yle ; et dans laquelle R⁴ est H ou un halogène ;
et au moins un excipient pharmaceutiquement acceptable, pour une utilisation comme inhibiteur de GPVI dans la prévention et/ou le traitement de la thrombose, ainsi que des pathologies associées à la formation d'un thrombus choisies parmi : accident ischémique cérébral, athérosclérose, maladie coronarienne ou infarctus du myocarde.

7. Composition pharmaceutique pour une utilisation selon la revendication 6, dans laquelle ledit composé de formule (I) est choisi dans le groupe constitué de Iₐ, I_{b}, I_{c}, I_{d}, Iₑ, I_{f} et Iₕ :

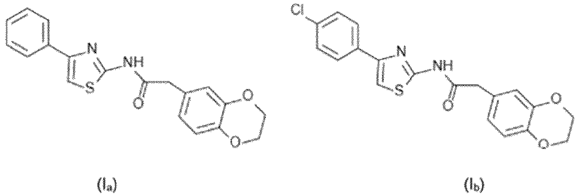

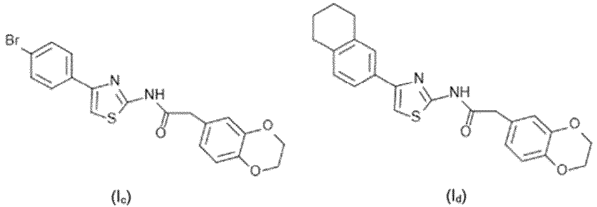

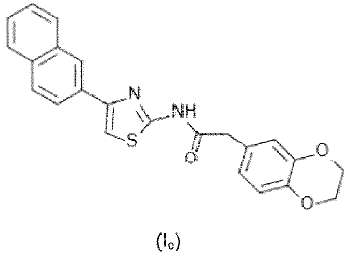

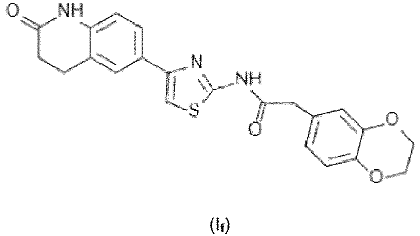

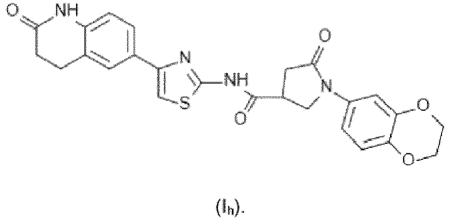

8. Composition pharmaceutique pour une utilisation selon l'une quelconque des revendications 6 ou 7, ladite composition pharmaceutique étant administrée avant, avec ou après l'administration d'un agent thérapeutique supplémentaire pour une utilisation dans la prévention et/ou le traitement de la thrombose.

9. Composition pharmaceutique pour une utilisation selon la revendication 8, ladite composition pharmaceutique comprenant en outre un autre agent thérapeutique supplémentaire pour une utilisation dans la prévention et/ou le traitement de la thrombose.

10. Composition pharmaceutique pour une utilisation selon l'une quelconque des revendications 8 ou 9, dans laquelle ledit agent thérapeutique supplémentaire est un médicament antiplaquettaire.

11. Composition pharmaceutique pour une utilisation selon l'une quelconque des revendications 8 ou 10, dans laquelle ledit agent thérapeutique supplémentaire est choisi dans le groupe constitué d'un inhibiteur de la cyclooxygénase, d'un inhibiteur du récepteur de l'adénosine diphosphate, d'un inhibiteur de la phosphodiestérase, d'un antagoniste du récepteur-1 activé par la protéase, d'un inhibiteur de la glycoprotéine IIB/IIIA, d'un inhibiteur de la recapture de l'adénosine et d'un inhibiteur du thromboxane.

12. Composition pharmaceutique pour une utilisation selon l'une quelconque des revendications 6 à 11, ladite composition pharmaceutique étant administrée par voie orale, transdermique ou parentérale.

13. Utilisation d'un composé de formule (I) dans un système de test *in vitro* pour la détection de l'activation plaquettaire médiée par GPVI.
